# EUROPEAN PATENT SPECIFICATION

(11) **EP 3 116 991 B1**
(45) Date of publication and mention of the grant of the patent: **23.01.2019**
(21) Application number: 15762448.7
(22) Date of filing: 11.03.2015
(51) Int. Cl.: C12N 5/074, C12N 5/10, C12N 5/0789, C12N 5/02

(54) **METHOD FOR INDUCING PLURIPOTENCY IN A HEMATOPOIETIC CELL**
VERFAHREN ZUR INDUZIERUNG VON PLURIPOTENZ BEI EINER HÄMATOPOIETISCHEN ZELLE
PROCÉDÉ D'INDUCTION DE PLURIPOTENCE DANS UNE CELLULE HEMATOPOÏÉTIQUE

(30) Priority: 11.03.2014 US 201461951051 P
(43) Date of publication of application: 18.01.2017
(73) Proprietor: Agency for Science, Technology and Research, Singapore 138632 (SG)
(72) Inventor: LOH, Yuin Han, Singapore 138632 (SG); TAN, Hong Kee, Singapore 138632 (SG)
(74) Representative: Mewburn Ellis LLP
(86) International application number: PCT/SG2015/050035
(87) International publication number: WO 2015/137885

(56) References cited:
- WO-A1-03/005909
- WO-A2-2011/159684
- RANDALL K MERLING ET AL: "Transgene-free iPSCs generated from small volume peripheral blood nonmobilized CD34 1 cells", BLOOD, vol. 121, no. 14, 5 February 2013 (2013-02-05), pages e98-e107, XP055339884, US ISSN: 0006-4971, DOI: 10.1182/blood-2012-03-420273
- LIN YE ET AL: "Blood Cell-Derived Induced Pluripotent Stem Cells Free of Reprogramming Factors Generated by Sendai Viral Vectors", STEM CELLS TRANSLATIONAL MEDICINE : SCTM, vol. 2, no. 8, 1 August 2013 (2013-08-01), pages 558-566, XP055338729, Durham ISSN: 2157-6564, DOI: 10.5966/sctm.2013-0006
- Hong Yu Chen ET AL: "Derivation of Transgene-Free Induced Pluripotent Stem Cells from a Single Drop of Blood : iPSCs Derivation from Blood Droplet" In: "Current Protocols in Stem Cell Biology", 17 August 2016 (2016-08-17), John Wiley & Sons, Inc., Hoboken, NJ, USA, XP055386756, ISBN: 978-0-470-15180-8 pages 4A.9.1-4A.9.10, DOI: 10.1002/cpsc.12, * the whole document *
- HONG-KEE TAN ET AL: "Human Finger-Prick Induced Pluripotent Stem Cells Facilitate the Development of Stem Cell Banking", STEM CELLS TRANSLATIONAL MEDICINE : SCTM, vol. 3, no. 5, 19 March 2014 (2014-03-19), pages 586-598, XP055386759, Durham ISSN: 2157-6564, DOI: 10.5966/sctm.2013-0195
- Anonymous: "A*STAR SCIENTISTS CREATE STEM CELLS FROM A DROP OF BLOOD", , 20 March 2014 (2014-03-20), XP055386762, Retrieved from the Internet: URL:https://www.a-star.edu.sg/Portals/0/me dia/Press Release/IMCB fingerprick iPSC_final.pdf [retrieved on 2017-06-30]
- MERLING, R.K. ET AL.: 'Transgene-free iPSCs generated from small volume peripheral blood nonmobilized CD 34+ cells' BLOOD. vol. 121, no. 14, 05 February 2013, pages E98 - E107, XP055339884
- SEKI, T. ET AL.: 'Generation of induced pluripotent stem cells from a small amount of human peripheral blood using a combination of activated T cells and Sendai virus' NATURE PROTOCOLS. vol. 7, no. 4, 2012, pages 718 - 728, XP055223860
- AREMAN, E.M. ET AL.: 'Ammonium Chloride Lysis for Red Blood Cell (RBC) Depletion of Peripheral Blood Stem Cell (PBSC) Products' ASH ANNUAL MEETING ABSTRACTS vol. 96, no. 11, 2000, page 325, XP008184444
- MOQUET, J. ET AL.: 'Gamma-H2AX biodosimetry for use in large scale radiation incidents: comparison of a rapid '96 well lyse/fix' protocol with a routine method' PEERJ. vol. 2, 2014, page E282, XP055223866

## Description

### CROSS-REFERENCE TO RELATED APPLICATION

This application claims benefit of, and priority from, US provisional application No. 61/951,051, filed on March 11, 2014.

### FIELD OF THE INVENTION

The present invention relates to methods for inducing pluripotent stem cells from hematopoietic cells obtained from a blood sample.

### BACKGROUND

Induced pluripotent stem cells (iPSCs) are important sources for stem cells for use in treating disease, for research of developmental stages and for examining disease mechansisms, in place of embryonic stem cells. This is particularly important when studying human cells and disease mechanisms and treatments due to ethical concerns relating to the use of human embryos.

Human iPSCs (hiPSCs) were first generated by the overexpression of several transcription factors in dermal fibroblasts (Takahashi et al., 2007; Yu et al., 2007; Park et al., 2008). Like human embryonic stem cells (hESC), hiPSCs are capable of differentiation into functional cell lineages and therefore hold great potential for disease modeling, drug screening and future therapeutic medicine (Kondo et al., 2013; Park et al., 2008).

A recent study used human urine samples for derivation of epithelial-like cells which can be reprogrammed to pluripotency (Zhou et al., 2011). This method provides an unlimited source of cells which can be collected conveniently and non-invasively. Nevertheless, this approach requires the collection of large volume of urine samples resulting in logistic difficulties for downstream storage and transportation (Xue et al., 2013).

Blood is considered an ideal source of cells for reprogramming due to its abundance and accessibility. Progenitor blood cells from mobilized, bone-marrow and cord blood are highly amenable to reprogramming (Loh et al., 2009; Ye et al., 2009; Giorgetti et al., 2009; Haase et al., 2009). However, cytokine-induced mobilization may result in side effects (Sohn et al., 2002), bone-marrow harvesting is invasive, and cord blood is limited to individuals who have deposited their samples at birth.

In comparison, peripheral blood T-cells can be easily obtained from donors and reprogrammed with high efficiency (Loh et al., 2010; Seki et al., 2010; Staerk et al., 2010). However, T-cell derived hiPSCs carry pre-existing V(D)J rearrangement at the T-cell receptor (TCR) loci and it is unclear whether these hiPSCs can differentiate normally or if such cells demonstrate tendencies for development into T-cell lymphomas (Serwold et al., 2007).

Recent studies report the reprogramming of non T-cell populations in peripheral blood. The reprogramming efficiencies in these studies are generally low, necessitating the addition of more factors (5-7 factors in some cases, including SV40LT, *TP53* shRNA) in the reprogramming protocols (Chou et al., 2011; Mack et al., 2011; Okita et al., 2013).

WO 03/005909 A1 describes a blood specimen sampling and insulated shipping kit that provides a user with materials that are needed to initiate blood flow, direct a blood sample into a sample vial, insulate the vial from heat and cold, protect the vial from impact, and ship the blood sample containing vial to a clinical laboratory for testing.

### SUMMARY

The present invention relates to methods for obtaining iPSCs from a small volume of peripheral blood.

The methods require only small blood volumes, for example as little as 10 µl of blood, due to the exclusion of polysaccharide preparations that are typically used to separate blood samples into various components.

Thus, the blood sample may be obtained by finger-prick. Finger-prick samples can be easily and conveniently collected without the need for trained phlebotomists, including by the subject at home or in another non-laboratory setting.

The use of Sendai virus to transfect CD71+ cells with pluripotency protein factors can provide iPSCs that are transgene free and that do not contain genomic rearrangement.

The methods may provide efficient rates of inducing pluripotency in an expanded finger-prick blood cell population. For example, using the method, finger-prick derived hiPSCs were generated from human peripheral blood at very high efficiency (100-600 colonies/mL of blood).

The ease of obtaining blood samples and the efficient rate of inducing pluripotency may allow for development of international hiPSC banking from large cohorts of donors.

In one aspect, there is provided a method of inducing pluripotency in a hematopoietic cell, as set out in claim 1.

The sample may be stored for up to 48 hours prior to use in the method, at a temperature of 3°C to 5°C or on ice.

The volume of the blood sample may be about 250 µl or less, including for example, from 10 µl to 20 µl.

Also disclosed herein is a method of inducing pluripotency in a hematopoietic cell, the method comprising: providing a blood sample collected from a subject in the absence of any polysaccharide preparation used for separating blood components, the blood sample having a volume of from about 10 µl to about 20 µl and being a sample previously collected by the subject by finger prick in a non-laboratory setting and which has been stored for up to about 48 hours, at a temperature of about 3°C to about 5°C or on ice; depleting the sample of red blood cells by treating the sample with a hypotonic erythrocyte lysis buffer in the absence of any polysaccharide preparation used for separating blood components, to obtain a remaining cell population; expanding the remaining cell population in the sample in a hematopoietic expansion medium to obtain an expanded cell population that contains CD71+ cells; and culturing the expanded cell population in the presence of Oct4, Sox2, and Klf4, and optionally c-MYC, in human embryonic stem cell medium to induce pluripotency.

As described herein, the hematopoietic cell may be a non-human hematopoietic cell, or may be a human hematopoietic cell.

The hypotonic lysis buffer may comprise ammonium chloride.

Expanding of the remaining cell population in the sample may be performed in a serum-free hematopoietic expansion medium. As well, the expanding may occur for a period of 12 to 16 days.

The resulting expanded cell population may, prior to the culturing to induce pluripotency, comprise 20 000 cells or greater. The expanded cell population prior to the culturing to induce pluripotency, contains 50 % or greater CD71+ cells.

Culturing to induce pluripotency may be performed in the presence or absence of feeder cells. In some embodiments, the Oct4, Sox2 and Klf4, and the optional c-MYC, may be included in the human embryonic stem cell medium during the culturing. In some embodiments, the expanded cell population may be transfected or transduced the with one or more nucleic acid vectors encoding the Oct4, Sox2 and Klf4, and the optional c-MYC during the culturing. For example, the one or more nucleic acid vectors may be viral vectors, episomal vectors, minicircle vectors, or synthesized mRNA. Viral vectors that may be used include adenoviral vectors, baculoviral vectors or Sendai viral vectors. In some embodiments, Sendai viruses are used to transduce the expanded cell population, and, for example, the expanded cell population may be exposed to the one or more Sendai viruses over a period of 24 hours, including for example at an MOI of 10 for each of the one or more Sendai viruses.

Once obtained, the resulting induced pluripotent cells may be stored in a cell bank or library.

Additionally, a portion of the originally collected blood sample may be, prior to the depleting, reserved for characterization, for example DNA sequencing and/or blood serotyping.

Also disclosed herein is a kit for collecting a finger prick blood sample for use in inducing pluripotency in a hematopoietic cell, the kit comprising a finger-prick device, a collection tube, a thermo-box, and instructions for collecting the blood sample. The kit may also include a shipping box or packaging for delivering the sample once collected, and/or one or more blood analysis tests.

### BRIEF DESCRIPTION OF THE DRAWINGS

The figures, which illustrate, by way of example only, embodiments of the present invention, are as follows.

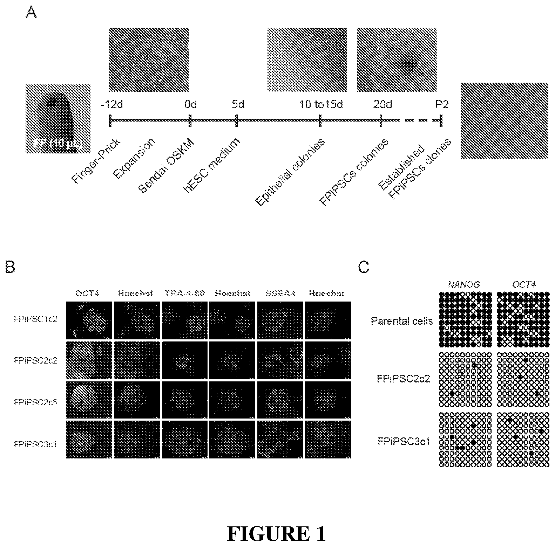

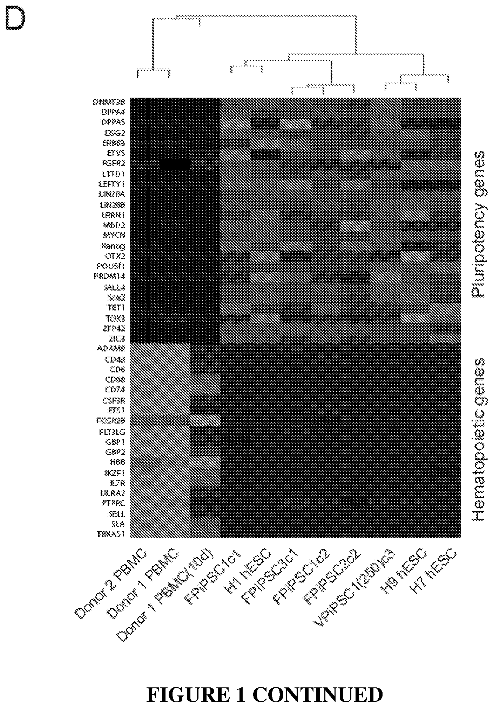

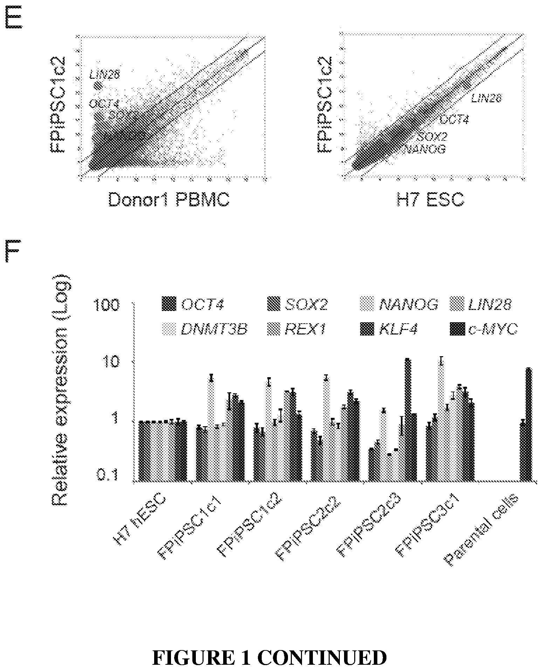

**Figure 1****. Derivation of hiPSCs from finger-prick blood samples.** (A) Schematic drawing of the strategy used for reprogramming human finger-pricked blood. (B) Immuno-fluorescent staining of FPiPSC colonies expressing pluripotency markers for TRA-1-60, SSEA4 and OCT4. (C) Bisulfite DNA methylation analysis on the promoter of *OCT4* and *NANOG* in FPiPSCs and in their parental cells. (D) Dendrogram showing un-supervised hierarchical clustering of the global expression profiles of hESCs, hiPSCs and parental blood cells (Top). Heat-map analysis of the hESCs, hiPSCs and parental blood cells for expression of Pluripotency and Hematopoietic specific marker genes (Bottom). (E) Scatter plots comparing FPiPSCs global gene expression profiles to parental cells (left) and H7 human ESCs (right). (F) qRT-PCR analyses for the expression of ESC marker genes *OCT4, SOX2, NANOG, LIN28, DNMT3B, REX1*, *KLF4* and *c-MYC* in FPiPSCs, H7 ESCs and parental cells.

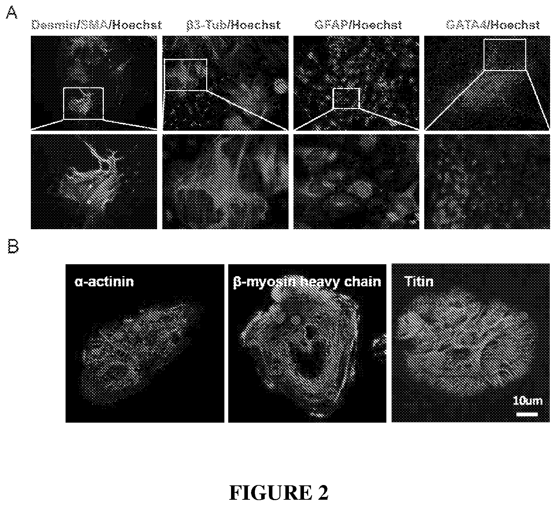

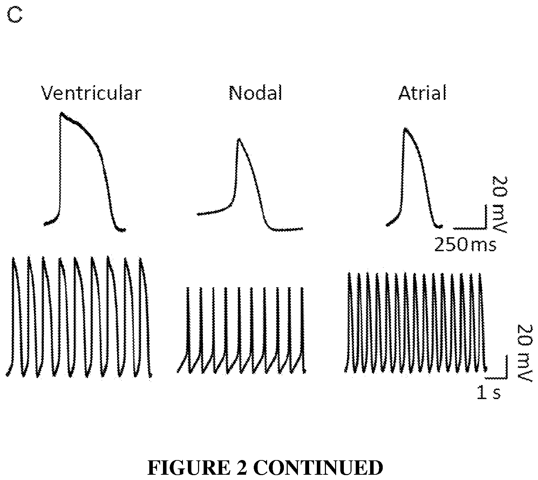

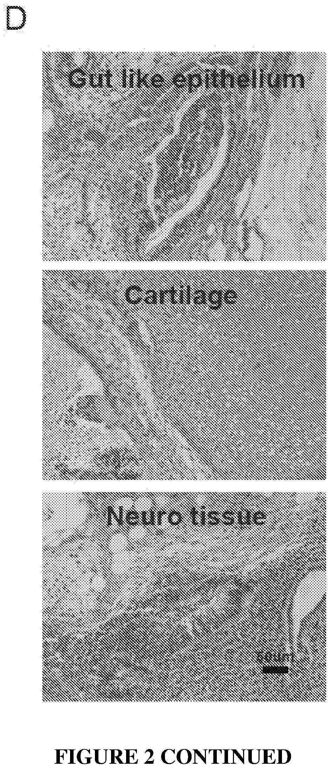

**Figure 2****. FPiPSCs differentiate into derivatives of three germ layers.** (A) Immuno-fluorescent staining of differentiation markers after *in vitro* differentiation of FPiPSCs. (B) Immuno-fluorescent staining of α-actinin, β-myosin heavy chain (β-MHC) and titin in hiPSCs derived cardiomyocyctes. (C) Patch clamp analyses of hiPSCs derived cardiomyocyctes. (D) Hematoxylin and eosin staining of teratomas derived from immune-deficient mice injected with FPiPSCs.

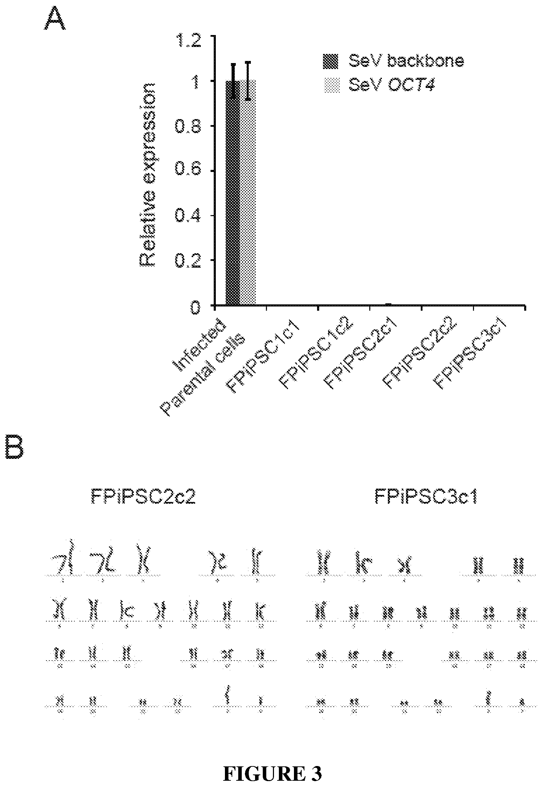

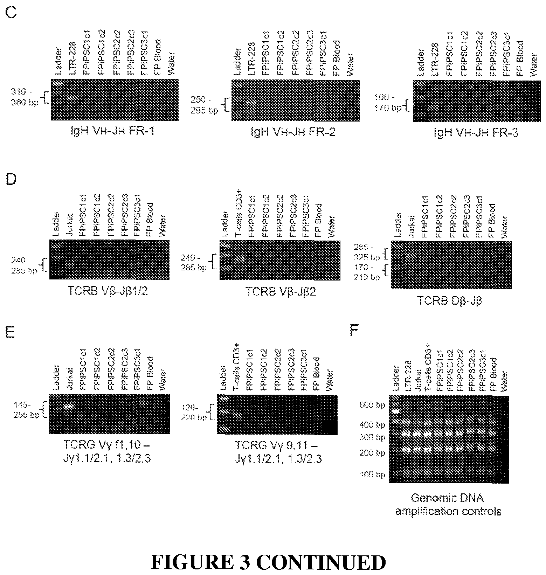

**Figure 3****. Characterizations of the FPiPSCs.** (A) Transgene expression analysis of the FPiPSC lines derived. (B) Cytogenetic analysis on FPiPSCs. (C) IgH rearrangement analysis on the FPiPSCs clones. (D) TCRB rearrangement analysis on the FPiPSCs clones. (E) TCRG rearrangement analysis on the FPiPSCs clones. (F) The genomic DNA amplification control experiment performed on samples used in the V(D)J rearrangement analyses.

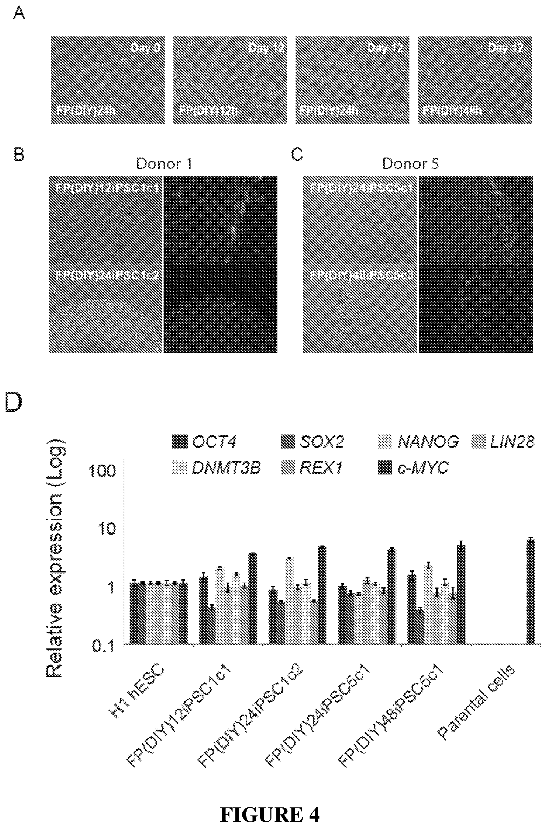

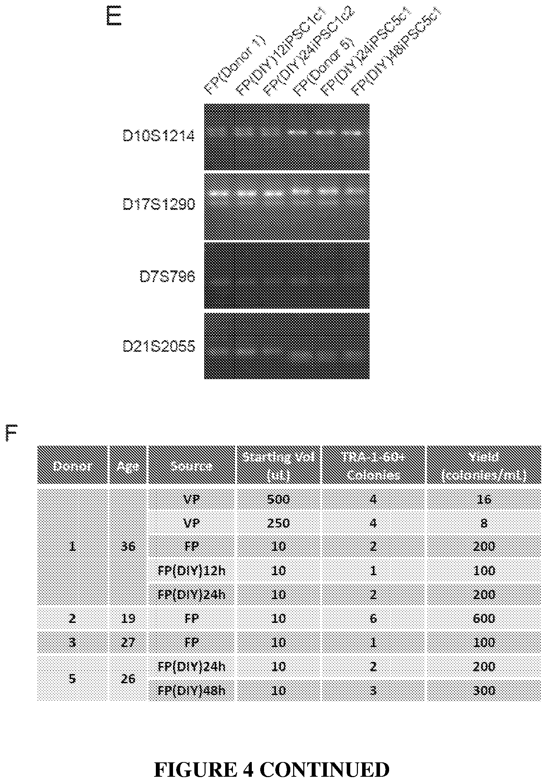

**Figure 4****. DIY concept for FPiPSCs generation.** (A) FP blood samples collected 12 hours, 24 hours and 48 hours prior to cell culture. (B) hiPSC colonies derived from Donor 1 DIY FP blood (12 hours and 24 hours) stained positive for a pluripotency marker, TRA-1-60. (C) hiPSC colonies derived from Donor 5 DIY FP blood (24 hours and 48 hours) stained positive for a pluripotency marker, TRA-1-60. (D) qRT-PCR analyses for the expression of ESC marker genes *OCT4, SOX2, NANOG, LIN28, DNMT3B, REX1*, and *c-MYC* in FP(DIY)iPSCs, H1 ESCs and parental cells. (E) DNA fingerprinting analysis confirms that FPiPS cell lines are derived from their parental lines. (F) Summary of hiPSCs derived from donors.

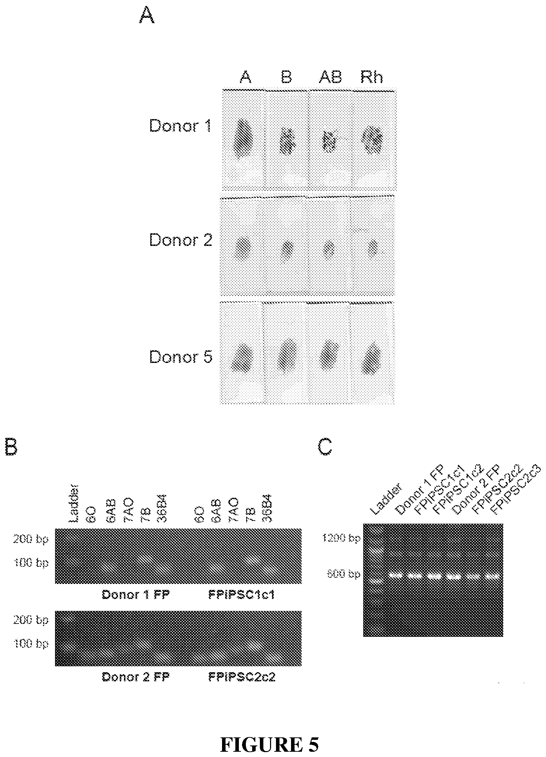

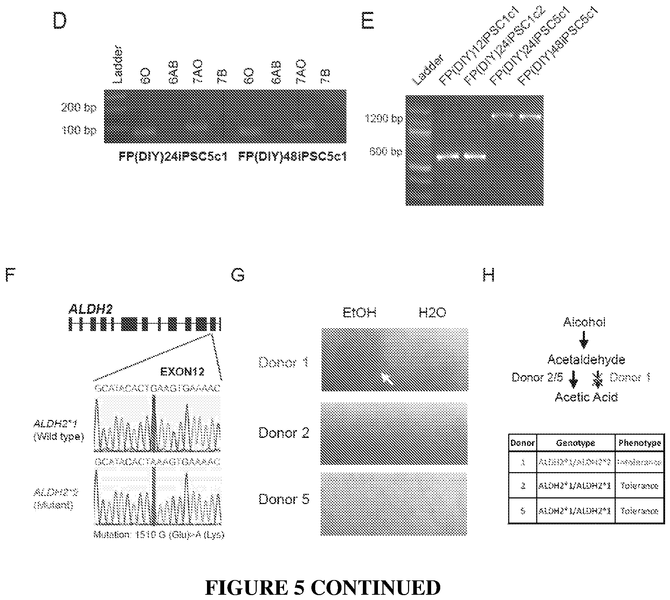

**Figure 5****. Blood typing and DNA genotype the finger-prick samples.** (A) Blood grouping of the donors from finger-prick samples. (B) Genotyping of the donors' blood group from finger-prick blood and derived hiPSCs. (C) Rhesus D antigen genotyping of the finger-prick samples and derived hiPSCs. (D) Genotyping of the donors' blood group from DIY finger-prick blood derived hiPSCs. (E) Rhesus D antigen genotyping of the DIY finger-prick samples derived hiPSCs. (F) SNP on the *ALDH2* loci. (G) Ethanol patch test on the donors. (H) The metabolic pathway for degradation of ethanol in the body and genotype/phenotype correlation for ALDH2.

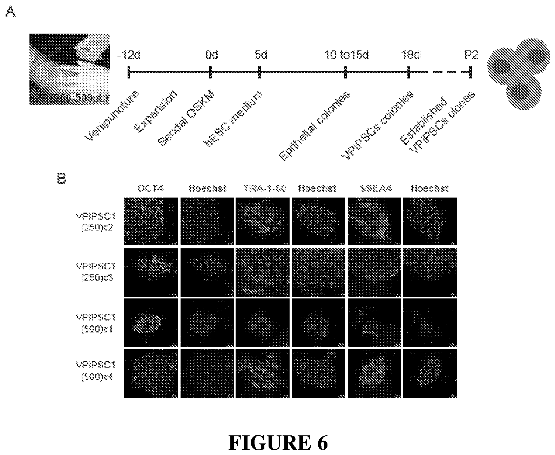

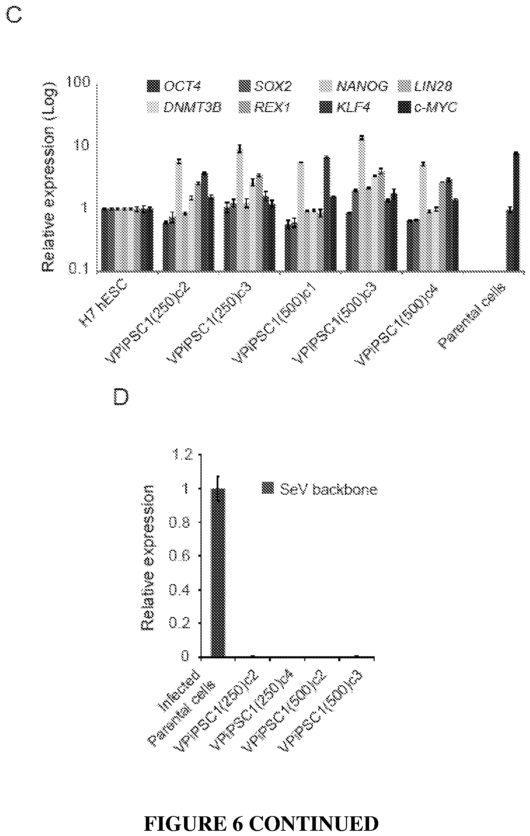

**Figure 6****. Derivation of Venipuncture (VP) Blood hiPSCs.** (A) Schematic drawing of the timeline for VP blood reprogramming. (B) Immuno-fluorescent staining of pluripotency markers on VPiPSCs. (C) Pluripotency gene expression analysis on VPiPSCs. The pluripotency genes examined are listed on the upper panel of the figure. (D) Transgene expression analysis of the VPiPSC lines after 4 passages.

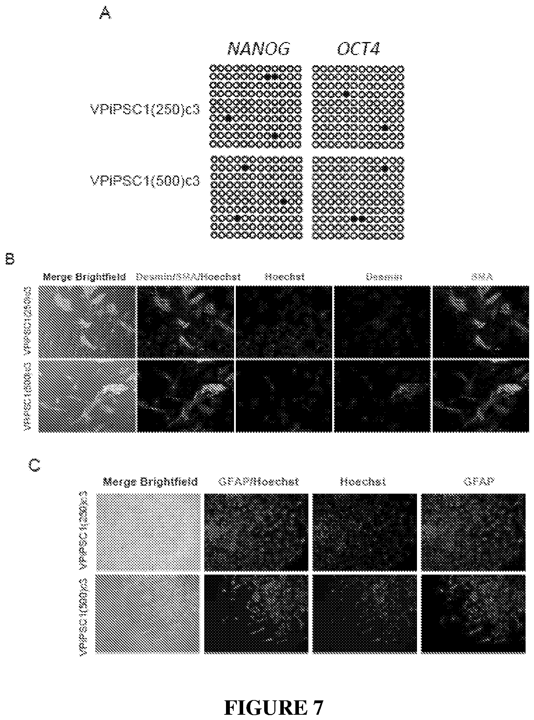

**Figure 7****. Characterization of VPiPSCs.** (A) DNA methylation analysis on the promoter of *OCT4* and *NANOG* in the VPiPSCs. The white and black circles represent unmethylated and methylated CpG islands in the promoter region, respectively. (B) Immuno-fluorescent staining of mesoderm markers, Desmin and SMA, of *in vitro* differentiated VPiPSCs. (C) Immuno-fluorescent staining of differentiation markers after *in vitro* differentiation of VPiPSCs. Ectoderm was stained by GFAP antibodies.

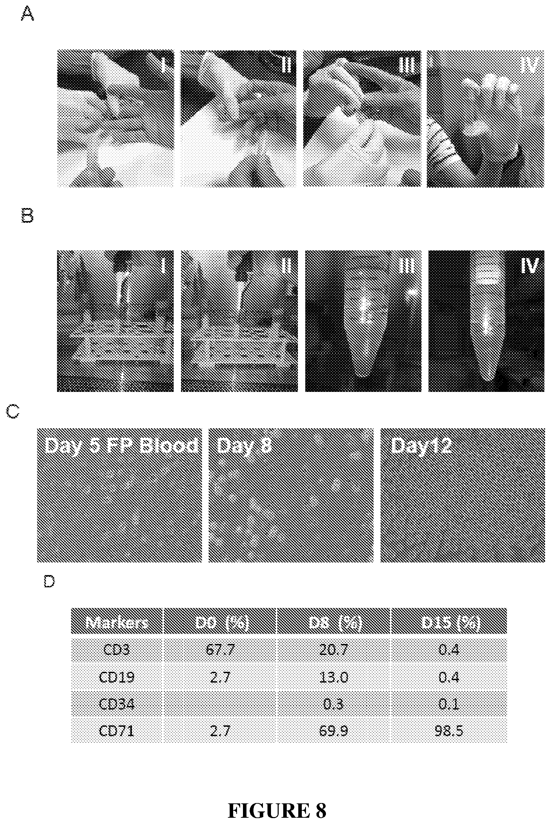

**Figure 8****. Isolation and Expansion of FP Blood Cells.** (A) Collection of FP blood. The figures are shown in a sequential manner (I to IV). (B) RBC depletion of FP blood. (C) Images of the purified FP blood cells from 5-12 days. All images were acquired with 20x objectives. (D) Cells were analyzed by flow cytometry to determine the cell-surface immunophenotype on specific days of culture (Day 0, 8, 15). Percentage of cells with cell-surface immunophenotypes, CD3(+), CD19(+), CD34 (+) or CD71(+) are summarized and represented in the table.

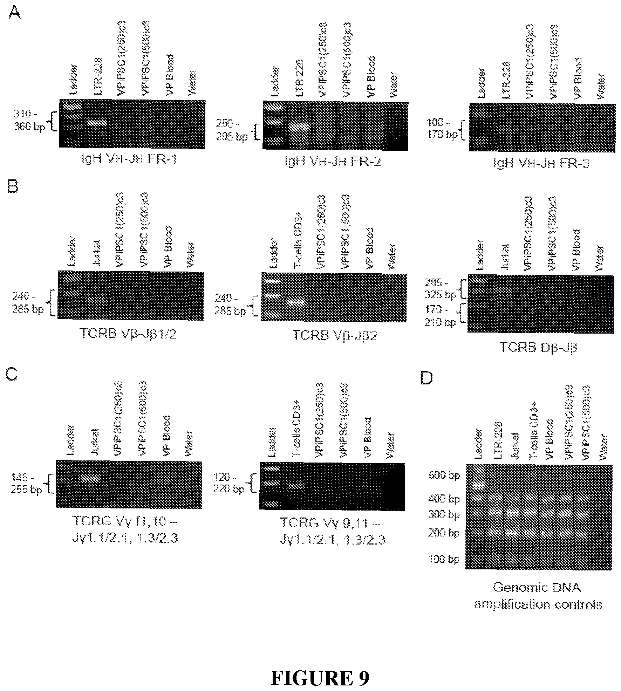

**Figure 9****. V(D)J Rearrangement of VPiPSCs Derived.** (A), (B) and (C) show the IgH, TCRB and TCRG rearrangement analysis on VPiPSCs derived. (D) Amplification control on VPiPSCs genomic DNA which was used in the analysis. In all figures, the expected PCR product ranges are stated on the left and the target regions are indicated below the gel images.

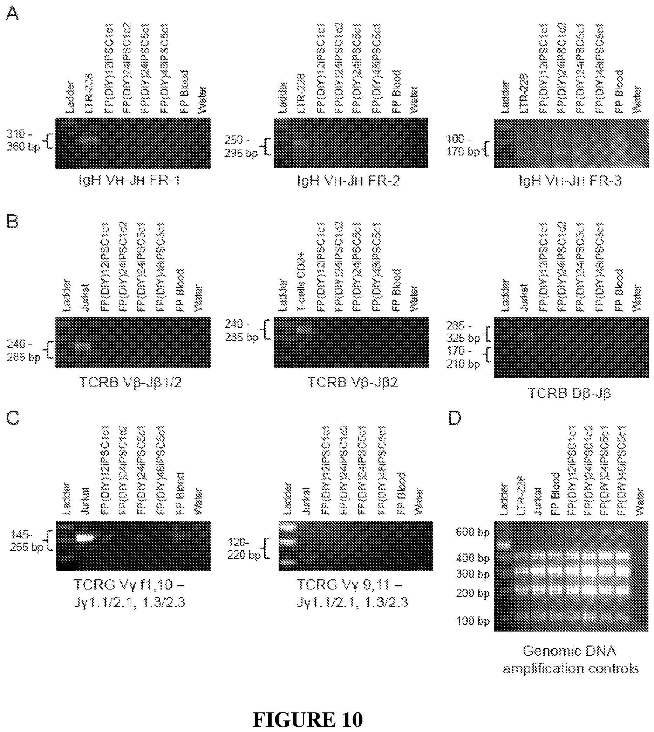

**Figure 10****. V(D)J Rearrangement of FP(DIY)iPSCs Derived.** (A), (B) and (C) show the IgH, TCRB and TCRG rearrangement analysis on FP(DIY)iPSCs derived. (D) Amplification control on FP(DIY)iPSCs genomic DNA which was used in the analysis.

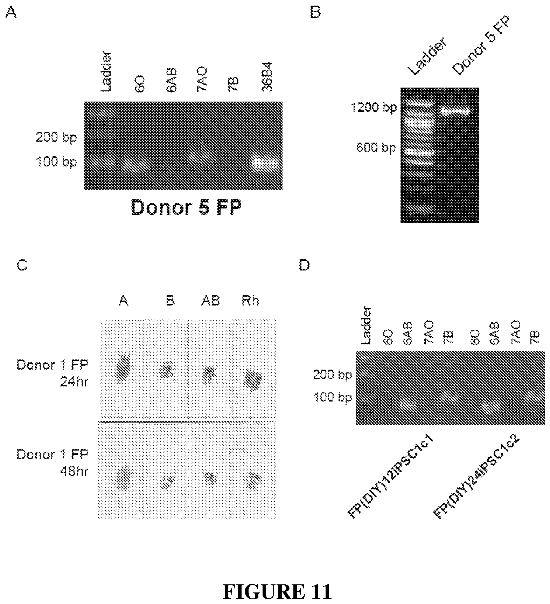

**Figure 11****. Blood typing and DNA genotyping of DIY Finger-prick blood and derivative iPSCs** (A) Genotyping of the Donor 5 blood group from DIY finger-prick samples. (B) Rhesus D antigen genotyping of Donor 5 DIY finger-prick samples. (C) Blood grouping of Donor 1 DIY finger-prick samples obtained 24 hours and 48 hours prior to analysis. (D) Genotyping of the donors' blood group from DIY finger-prick blood derived hiPSCs.

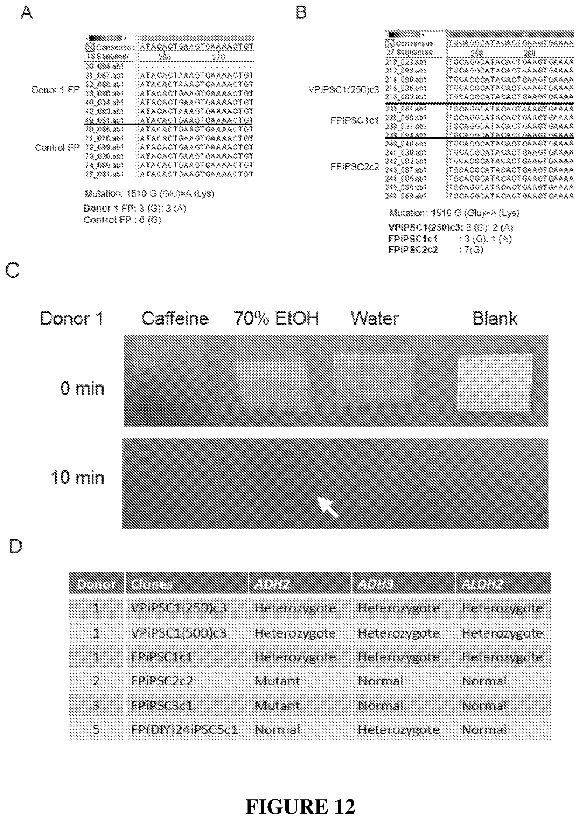

**Figure 12****. Genotyping of *ALDH2, ADH2 and ADH3* on hiPSCs derived.** (A) and (B) showed the sequencing alignment results on *ALDH2* of Donor 1 and Donor 2 FP blood (A) and their respective hiPSCs (B). (C) Ethanol patch test on Donor 1. Caffeine, 70% ethanol, water and a blank patch were applied on Donor 1's skin (0 min). After 10 minutes, the region which was applied with 70% ethanol showed flush reaction as indicated by the white arrow. (D) Summary of genotyping *ADH2, ADH3* and *ALDH2* on FPiPSCs.

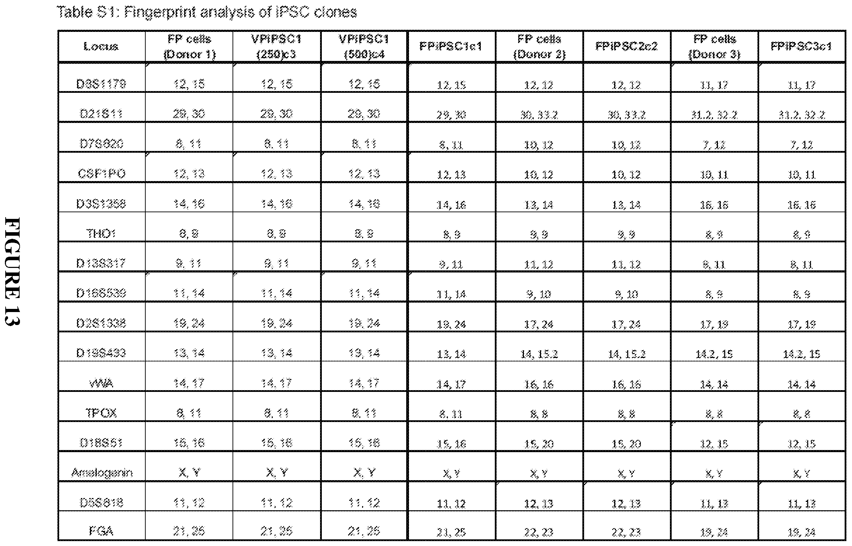

**Figure 13****. Contains Table 1: Fingerprint analysis data of iPSC clones.**

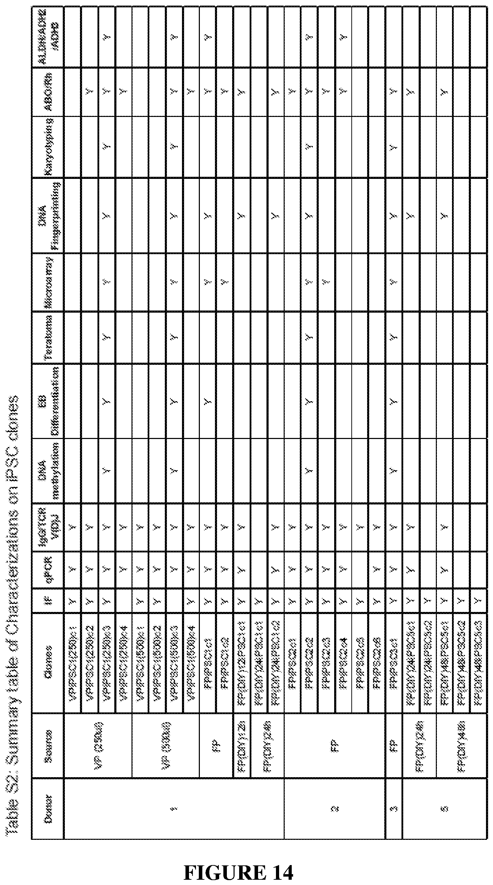

**Figure 14****. Contains Table 2: Summary of Characterizations performed on iPSC clones.**

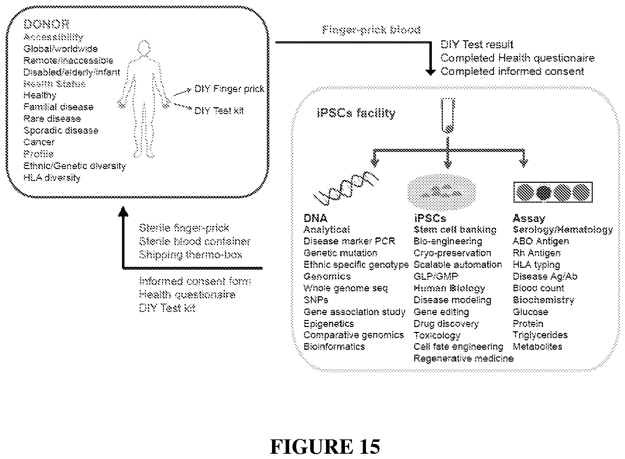

**Figure 15****. Schematic depiction of an integrative strategy for hiPSCs banking.** Illustration of an integrative strategy for hiPSCs banking using the method of the invention to derive hiPSCs.

### DETAILED DESCRIPTION

The methods as described herein relate to collection of a blood sample containing peripheral blood cells that are non T-cells and which can be reprogrammed into pluripotent cells. The sample is collected under conditions free from polysaccharide additives or preparations, such as hydrophilic polysaccharides that are typically added to collected blood samples to assist with centrifugation separation of blood components within the sample. The sample is treated with a hypotonic erythrocyte lysis buffer to remove red blood cells, prior to expansion of cells and and reprogramming to obtain iPSCs. Due to the absence of the polysaccharide preparations under the collecting conditions, and due to the use of the ethrocyte lysis buffer, very small volumes of blood may be used for reprogramming, including as little as 10 µl of blood volume.

Prior to the development of the methods described herein, previous studies reported the reprogramming of non T-cell populations in peripheral blood. However, the reprogramming efficiencies in these studies are generally low, necessitating the addition of more factors (5-7 factors in some cases, including SV40LT, *TP53* shRNA) in the reprogramming protocols (Chou et al., 2011; Mack et al., 2011; Okita et al., 2013).

To date, all described studies for reprogramming of peripheral blood cells require a considerable amount of starting material (about 10 mL), which are obtained via venipuncture performed by experienced phlebotomists. Such requirements make sample collection, transport and storage inconvenient. Furthermore, if samples are to be collected for inclusion in hiPSC banks, large scale collection of samples will be required, and previously described collection and reprogramming methods could limit the recruitment of large numbers of potential donors. Two studies described the generation of hiPSCs from a relatively small volume of peripheral blood; however, in these studies, from 2 mL to 6 mL of peripheral blood was still needed in order to purify enough CD34+ cells for reprogramming (Merling et al., 2013; Ye et al., 2013).

Thus, in one aspect, there is provided a method of inducing pluripotency in a hematopoietic cell as set out in the claims. The method is conducted *in vitro* on cells obtained from a peripheral blood sample collected from a patient in a manner as as set out in the claims.

Inducing pluripotency in a hematopoietic cell refers to converting the hematopoietic cell, which is non-pluripotent, to a pluripotent cell. As used herein, an iPSC is a pluripotent stem cell that has been induced to a pluripotent state from a non-pluripotent originator cell, for example a partially or fully differentiated cell that can be induced to become pluripotent by exposure to appropriate conditions and transcription factors or other protein factors that regulate gene expression profiles in pluripotent cells. The iPSC is thus a pluripotent cell that has been derived from a non-pluripotent originator cell and is not an embryonic stem cell.

Thus, an iPSC is fully undifferentiated and can potentially be differentiated into any type of cell, for example a cell from one of the three germ layers, the mesoderm, the endoderm, and the ectoderm, and also for example partially differentiated or fully differentiated cells of any particular lineage, for example bone cells, neural cells, skin cells, muscles cells, or any partially differentiated precursor cells of such cell types. An iPSC can be identified by its expression of pluripotency markers, for example, expression of one or more of OCT4, TRA-1-60, SSEA-4, SOX2, KLF4, c-MYC, REX1, NANOG, LIN28, and DNMT3B.

The blood sample collected is a peripheral blood sample, meaning it is collected from circulating blood and contains circulating blood cells including erythrocytes, leukocytes, and platelets, as well as circulating hematopoietic cells. As used herein, reference to a hematopoietic cell is reference to a blood precursor cell that can differentiate to become the various types of red blood cells, and which may typically express the CD71 cell surface marker.

As used herein, the term "cell' refers to and includes a single cell, a plurality of cells or a population of cells where context permits, unless otherwise specified. Similarly, reference to cells also includes reference to a single cell where context permits, unless otherwise specified.

The hemapoietic cell or cells may be in an *in vivo* context prior to extraction from a subject, or may be in an *in vitro* context, including for example contained within an extracted sample, or may be contained within a culture, including batch culture or in tissue culture plates, and may be in suspension or may be attached to a culture support surface.

Similarly, once induced to become pluripotent, the iPSC(s) may be *in vitro* and may be contained within a culture, including batch culture or in tissue culture plates, and may be in suspension or may be attached to a culture support surface.

The subject from whom the blood is to be collected may be any animal, including any mammal, including a non-human mammal or a human. In some embodiments, the subject is a human subject.

The method involves use of a blood sample collected from the subject in the absence of any polysaccharide preparation such as that typically used when collecting blood in a laboratory setting.

Thus, the blood sample used in the method is collected in a manner that is free from polysaccharide preparations that are typically included with collected blood samples to assist with the separation of blood components. Typical laboratory practice when collecting a blood sample is to extract several milliliters of blood, for example about 10 to 20 ml, collecting the extracted blood in a tube that may already contain a polysaccharide preparation, or to which a polysaccharide preparation may subsequently be added after collection, followed by gradient centrifugation to separate the different blood components into layers. Polysaccharide preparations that are typically used for blood collection and separation are commercially available. Such polysaccharide preparations tend to contain hydrophilic polysaccharides, for example, heparin or the branched polysaccharide FICOLL™.

Thus, as used herein, reference to a blood sample that is free from a polysaccharide preparation, or that has been collected in the absence of a polysaccharide preparation, is reference to a blood sample that has been collected such that no polysaccharide preparation is present in the collection vessel and no polysaccharide preparation is subsequently added to the collected sample in order to assist with separation or isolation of blood components.

The volume of blood sample used in the methods is small, and thus the sample collected can be small. The volume of the blood sample used in the method is from 10 µl to 250 µl, as defined in the claims. As little as 10 µl may be used in the method to generate iPSC, and thus in some embodiments, the sample collected may be from 10 µl to 20 µl in volume, for example the volume of a typical drop of blood.

Although the blood is collected in the absence of any polysaccharide preparation, the blood may be collected in the presence of an anticoagulant, such as EDTA. The anticoagulant may be already present in the vessel or tube to which the blood is added, or may be subsequently added to the vessel shortly after the blood is added.

If an anticoagulant is used, sufficient anticoagulant should be added based on the volume of blood sample that is to be collected, so as to prevent the blood from coagulating prior to use in the method, but not so much as to result in cell lysis or to render any hematopoietic cells non-viable.

Since a small volume of blood is being collected, there is no need to have the blood extracted by syringe by a trained phlebotomist. Thus, the sample may be collected by pricking the pad of a finger or thumb of the subject, or any other location on the underside of the hand or foot, as such pricks are relatively low pain and the blood can easily be captured in a tube or other collection vessel. Since no syringe extraction is necessary, the sample does not need to be collected in a laboratory setting, and may, in some embodiments, be conveniently collected by the subject.

Once collected, the blood sample may be used directly in the method. Alternatively, the blood sample may be stored on ice or may be refrigerated at a temperature of 3°C to 5°C, or 4°C, prior to use in the method. Thus, the sample may be stored on ice for up to 8 hours, up to 12 hours, up to 16 hours, up to 20 hours, up to 24 hours, up to 28 hours, up to 32 hours, up to 36 hours, up to 40 hours, up to 44 hours, or up to 48 hours. If the sample is to stored longer than 48 hours, the sample can be frozen using liquid nitrogen and stored for future use.

The collected blood sample that is free from any polysaccharide preparation is treated with a hypotonic erythrocyte lysis buffer in order to deplete the sample of erythrocytes. Such lysis buffers are known, and may contain, for example, hypotonic lysis buffer may contain ammonium chloride.

For example, the blood sample may be mixed with 10 to 20 parts hypotonic erythrocyte lysis buffer. The treated sample may then be centrifuged to remove cell debris from the lysed red blood cells.

The treatment with hypotonic erythrocyte lysis buffer is also performed in the absence of any polysaccharide preparation. Thus, the resulting treated sample remains free from any polysaccharide preparation and has been depleted of red blood cells, leaving a remaining cell population that includes hematopoietic cells.

The remaining cell population that contains hematopoietic cells is then expanded to increase the total population of hematopoietic cells. As indicated above, hematopoietic cells may include CD71+ cells, and thus the remaining cell population is expanded to obtain an expanded cell population that contains CD71+ cells.

The cell expansion of the hematopoietic cells in the remaining cell population is performed using hematopoietic expansion medium. Such techniques are known and expansion media are commercially available. For example, StemSpan™ (Stemcell Technologies) may be used.

The expansion medium may contain other factors and supplements necessary to expand the remaining cell population contained within the remaining cell population of the sample, preferably without further differentiation of the cell population. For example, the expansion medium may be supplemented with amino acids, antioxidants or growth factors. As well, the medium may be supplemented with antibiotics or other components to prevent microbial contamination.

In some embodiments, the expansion medium may be serum free, and thus the expansion of the remaining cell population may be done in the absence of serum, including human or non-human serum.

For example, in some embodiments, the hematopoietic expansion medium used may be StemSpan™ Serum-Free Expansion Medium (09650, Stemcell Technologies) supplemented with 1 X Pen/Strep (Gibco), 1 X L-Glutamine (Gibco), 1 X NEAA (Gibco), 50 µg/mL L-ascorbic acid (Sigma), 50 ng/mL stem cell factor (Peprotech), 10 ng/mL interleukin-3 (Peprotech), 40 ng/mL insulin-like growth factor-1 (Peprotech), 2 U/mL erythropoietin (R&D Systems), 1 µM dexamethasone (Sigma), with or without 10 ng/mL of interleukin-6 (Peprotech).

The remaining cell population may undergo expansion in order to obtain a sufficient number of cells to allow for efficient induction of pluripotency. For example, the remaining cell population containing the hematopoietic cells including CD71+ cells may be expanded to obtain a total cell population of 20 000 cells or greater, 20 000 to 30 000 cells.

Depending on the growth conditions used and the expansion medium used, as well as the type of animal used as a subject, the length of time for expansion of the remaining cell population may vary. In some embodiments, the remaining cell population may undergo expansion for a period of up to 16 days, for example from 12 days to 16 days.

At the end of the expansion period, an expanded cell population is obtained. The proportion of the CD71+ cells present in the expanded cell population is at least half, in order to improve the efficiency of the induction of pluripotency. For example, upon completion of the expansion period, the expanded cell population may contain 75% or greater CD71+ cells, 80% or greater CD71+ cells, 85% or greater CD71+ cells, 90% or greater CD71+ cells, 95% or greater CD71+ cells, or 98% or greater CD71+ cells.

The expanded cell population containing the CD71+ cells is then induced to become pluripotent.

Methods of inducing pluripotency are known, for example as described in Takahashi and Yamanaka (2006) Cell 126:663-676.

The expanded cell population may be induced to become pluripotent by culturing the expanded cell population in the presence of pluripotency protein factors Sox2, Oct4, Klf4, and optionally c-Myc, in embryonic cell medium under conditions suitable for growth and that promote growth of embryonic stem cells. As used herein, reference to pluripotency protein factors is reference to Sox2, Oct4, Klf4, and optionally c-Myc.

Culturing the expanded cell population in the presence of the pluripotency protein factors includes contacting the cells with the various pluripotency protein factors so that the pluripotency protein factors are taken up by the cells, as well as transfecting or transducing the cells with nucleic acids encoding the various pluripotency protein factors and co-expressing the pluripotency protein factors.

Contacting the expanded cell population with the pluripotency protein factors may be achieved by including the pluripotency protein factors in the hematopoietic expansion medium at the end of the expansion period. The medium may be changed to embryonic stem cell medium, which may be from the pluripotency protein factors.

Alternatively, the expanded cell population may be transfected or transformed with one or more nucleic acid molecules encoding the pluripotency protein factors.

The one or more nucleic acid molecules encoding the pluripotency protein factors may be, for example, viral vectors, episomal vectors, minicircle vectors or synthesized mRNA. Viral vectors that may be used include adenoviral vectors, baculoviral vectors, lentiviral vectors or Sendai viral vectors.

In some embodiments, the one or more nucleic acid molecules are one or more Sendai viruses that encode the pluripotency protein factors. Such viruses are commercially available, for example as part of the CytoTune-iPS Reprogramming Kit™ (Life Technologies). The expanded cell population may be transduced by adding the appropriate Sendai viruses, at an MOI of about 10 for each virus, to the expansion medium. Following transduction, the medium may be changed for embryonic stem cell medium.

As will be appreciated, co-expression of the pluripotency protein factors will involve expression from a suitable promoter that is operably linked to the coding region for the particular pluripotency protein factor. A first nucleic acid sequence is operably linked with a second nucleic acid sequence when the sequences are placed in a functional relationship. For example, a coding sequence is operably linked to a promoter if the promoter activates the transcription of the coding sequence.

Once the cell has been contacted with the pluripotency protein factors, or once the cell has been transfected or transduced with nucleic acid molecules encoding each of the pluripotency protein factors and the cell has been exposed to conditions for co-expression, the cells may be cultured under conditions suitable for growth of embryonic stem cells.

Embryonic stem cell media are known and are commercially available. Such medium include, for example, DMEM/F12 (Gibco) or mTeSR1 (Stemcell Technologies), which may be further supplemented with any necessary factors or components. For example, the stem cell medium may be supplemented with serum, amino acids, antioxidants or growth factors. As well, the medium may be supplemented with antibiotics or other components to prevent microbial contamination. Generally, mTeSR1 is a defined medium that can be used for feeder-free culturing of hESC and iPSC. In some embodiments, mTeSR1 contains bovine serum albumin, rh bFGF, rh TGFβ, lithium chloride, pipecolic acid and GABA. Although the mTeSR1 defined medium may be commercially available, this medium is known and protocols are available that allow a skilled person to readily prepare mTeSR1 using routine laboratory methods.

In some circumstances, it may be desirable to use feeder cells to promote stem cell growth in culture, in keeping with standard embryonic stem cell culture techniques, for example mouse embryonic fibroblasts.

If preferred, individual colonies of induced pluripotent cells may be selected and then expanded in order to obtain a clonal population of an induced pluripotent cell, in accordance with standard cell culture techniques.

The method as described herein may induce iPSCs at high efficiency. As compared to the work by Merling and colleagues which described the isolation of 20 hiPSC colonies starting with 1 mL of human peripheral blood (Merling et al., 2013), the reprogramming yield of 100-600 colonies per mL in the present method using blood samples obtained by finger-prick is a significant improvement.

While the method provides the possibility of collecting small volume of DIY finger-prick samples taken by a subject at a remote location, this does not eliminate the benefits of collecting the samples in a hospital or laboratory setting. That is, when obtained in a medical setting, it may be possible to obtain more complete and/or accurate patient medical and diagnostic information.

As well, since only a small volume of blood is need to perform the method to obtain iPSCs from the blood sample, remaining blood from the sample, including a single drop of blood, may be used for other types of analysis. For example, the remaining volume of the blood sample not used in the method can be tested for disease genotype, or undergo deep genome sequencing to uncover an extensive catalogue of genetic variations, SNPs and haplotypes. Analysis of blood provides a general assessment of the donor's health and phenotype as the facility can perform serotyping, measure blood count and levels of various metabolites.

Thus, due to the small volume of blood need for the method, a single drop of finger-prick blood sample can be used for parallel experiments in cellular reprogramming, DNA sequencing and blood typing/biochemistry.

The use of finger-prick samples can aid the recruitment of donors from geographically, genetically and ethnically diverse populations, which may help to build a diverse iPSC bank. This method of obtaining iPSCs may be used in conjuction with recent advances in gene-editing (Cong et al., 2013), as well as automation and miniaturization of iPSCs derivation technologies (Geti et al., 2012). Thus, this method may increase the functionality, throughput and feasibility of operating a large scale international iPSCs bank.

The iPSCs obtained in the method can be used for stem cell banking, disease modeling, drug screening, toxicology tests and cell fate engineering. The method presents an opportunity for banking hiPSCs with full annotations of associated genotype (DNA sequencing), phenotype (Serological and DIY test) and clinical information (Blood biochemistry and Health questionnaires).

Numerous hiPSC banking initiatives are currently being established around the world (Rao et al., 2013; Turner et al., 2013). The potential of such stem cell repositories can only be realized if they have access to a wide range of donors/patients of diverse ethnicity, genotype and disease. The ability to reprogram from a small volume of finger-prick samples provides a strategy for hiPSC banks to access a large cohort of donors.

Using the current methods, the hiPSC facility could provide a package consisting of the DIY finger-prick collection kit, shipping thermo box, informed consent form, health questionnaire and DIY test kit to the donors. In particular, donors with difficulties in travelling due to logistics, financial or physical conditions (for example, the bed-ridden, elderly, disabled and infants) will benefit from this strategy. It will also be possible to recruit donors with rare disease phenotypes from remote locations.

Thus, a kit or commercial package is also contemplated. The kit may contain a finger-prick device, a collection tube, a thermo-box, instructions, and optionally a shipping box or packaging for delivering the sample once collected. The kit may further include an informed consent form, and/or a health questionnaire, and/or various blood analysis test kits to allow for DIY analysis such as sugar levels, blood typing, etc.

Upon receipt of the DIY kit, the donor can perform a finger-prick to collect blood samples in a tube containing anticoagulant and store it at about 4°C or so. The health questionnaires may help to detail the clinical conditions and medical history of the donors. Lastly, the donors can perform specific DIY blood tests, such as measurement of sugar level. These tests can be tailored according to specific questions that the study or facility aims to address. The completed documents, consent form and finger-prick samples can simply be returned to the hiPSC bank via global courier services.

The methods, cell populations, kits, and uses as described herein are further exemplified by way of the following non-limiting examples.

### EXAMPLES

### EXAMPLE 1

Efficient derivation of transgene-free hiPSCs from human finger-prick blood is described. Single drop volumes of finger-prick samples were found to be sufficient for performing cellular reprogramming, DNA sequencing and blood serotyping in parallel.

### Materials and Methods

***Finger-pricked and venous blood samples.*** 10 µL of finger-tip capillary blood was collected in a sterile laboratory setting. The samples were lysed in 2 mL of 1x RBC lysis buffer (00-4300-54, eBioscience) for 10 minutes before spinning at 250g for 5 minutes. The lysis buffer was aspirated immediately after the centrifugation. Purified cells were resuspended with 500 µL of cell expansion medium and seeded into one well of 24-well tissue culture plate (3536, Corning). For the DIY experiment, the donors were asked to perform a finger-prick themselves and to collect the blood into a Microtainer tube containing anti-coagulant ((422)365974, Becton Dickinson). The tube can be pre-sterilized over flame or under UV illumination. The DIY blood samples were stored on ice and RBC lysis was performed 12, 24 or 48 hours later. The FP blood cell expansion medium (Chou et al., 2011; van den Akker et al., 2010) contained StemSpan™ Serum-Free Expansion Medium (09650, Stemcell Technologies) supplemented with 1 X Pen/Strep (Gibco), 1 X L-Glutamine (Gibco), 1 X NEAA (Gibco), 50 µg/mL L-ascorbic acid (Sigma), 50 ng/mL stem cell factor (Peprotech), 10 ng/mL interleukin-3 (Peprotech), 40 ng/mL insulin-like growth factor-1 (Peprotech), 2 U/mL erythropoietin (R&D Systems), 1 µM dexamethasone (Sigma), with or without 10 ng/mL of interleukin-6 (Peprotech). Medium was changed every day by carefully pipetting out half of the medium and replacing with fresh medium. 12 to 16 days later, when the cell population reached 20-30K in numbers, they were transduced with Sendai virus. For VPiPSC derivation, 250 µL or 500 µL of peripheral blood was collected through venipuncture. PBMCs was purified using Ficoll-Paque PLUS (ρ=1.077±0.001 g/mL) (17-1440-03, GE Healthcare) according to manufacturer's instructions. The cells were then cultured as described for finger-prick samples. The use of finger-prick blood samples was approved by the ethics committee of the National University of Singapore. Written informed consent was obtained from all donors.

***Cellular reprogramming.*** 20-30K of cells were transduced by *OCT4, SOX2, KLF4,* and *c-MYC* Sendai Virus (CytoTune™-iPS Reprogramming Kit, Life Technologies) with each factor at MOI=10 (approximately 5 µL of each factor) (Yang et al., 2012). The transduction was terminated after 24 hours by replacing with fresh cell expansion medium. At Day 3, cells were transferred to 4 or 5 wells of irradiated CF1-MEFs (seeded at density of 200K per well) in 6-well tissue culture plates (3516, Corning) and cultured with 1:1 ratio of expansion and hESC medium (DMEM/F12 (Gibco) supplemented with 20% Knockout Serum Replacement (Gibco), 100 µM MEM non-essential amino acid solution (Gibco), 100 µM β-mercaptoethanol (Gibco), 1 X Penicillin-Streptomycin (Gibco), 1 X L-Glutamine (Gibco) and 10 ng/mL bFGF (Gibco)). 2 days later, the medium was changed to hESC medium with daily media change. From Day 14, reprogramming continued with MEF-conditioned hESC medium and mTeSR1 (Stemcell Technologies) in a 1 to 1 ratio. The volume of medium used for 6-well culture was 2 mL per well. Once hiPSC colonies resembling hESCs in morphology emerged, the colonies were mechanically picked and replated onto MEFs for expansion.

***Gene expression analysis.*** Direct application of PureZol (Bio-rad) onto adherent cells or cell pellets was performed for RNA isolation. RNA clean-up was performed using a QIAGEN RNeasy Kit. First strand cDNA synthesis was performed using iScript cDNA synthesis kit (Bio-Rad). Quantitative PCR analyses were performed using the CXF384 Realtime System (Bio-Rad) and KAPA SYBR FAST qPCR Master Mix (2X) (KK4602).

***Immuno-histochemistry staining.*** Cells were fixed in 4% paraformaldehyde for 15 minutes. For non-surface marker staining, the cells were permeabilized with 0.1% Triton X-100 for 30 minutes, and blocked in PBS containing 8% FBS for 30 minutes. Cells were incubated with primary antibodies overnight at 4°C, washed, and incubated with Alexa Fluor (Invitrogen) secondary antibodies for 2 hours or incubated with fluorophore conjugated antibodies for 1 hour. The cells were stained by Hoechst 33342 for 30 minutes before imaging. SSEA-4 Alexa 647 (560219) and TRA-1-60 Alexa 647 (560173) antibodies were obtained from Becton Dickinson Biosciences. OCT3/4 antibodies were obtained from Abcam (ab19857). Antibodies against GFAP were obtained from Sigma (G9269); anti-β3-tubulin from Covance (MMS-435P); anti-GATA4 from Santa Cruz Biotechnology (Sc-25310); anti-SMA from Pierce (MA1-06110) and anti-Desmin from Cell Signalling (5332S). For live staining, StainAlive™ TRA-1-60 Antibody (DyLight™ 488) (09-0068, Stemgent) was used according to manufacturer's protocol.

***Bisulfite genomic sequencing.*** Bisulfite treatment of genomic DNA (gDNA) was carried out using a CpGenome DNA Modification Kit (Chemicon) according to the manufacturer's protocol. Briefly, converted gDNA was amplified by PCR *OCT4* primer set 3, as reported previously (Freberg et al., 2007). PCR products were gel-purified and cloned into pCR2.1- TOPO using the TOPO TA cloning kit (Invitrogen). Bisulfite conversion efficiency of non-CpG cytosines ranged from 80% to 99% for all individual clones for each sample. Every clone of each sample was verified by sequencing with the M13 primers.

***Flow cytometry.*** The blood cells were stained with a panel of antibodies at the manufacturer's suggested dilution in PBS at 4°C for 20 minutes. This panel included APC-CD3, PE-CD34 and APC-CD19 from BD Bioscience and APC-CD71 from Miltenyi Biotech. Propidium iodide (Sigma) was used for live/dead discrimination except of samples stained with PE-CD34. Flow cytometry was performed on a FACS Canto II machine (BD Science), and data were analyzed using FACS Diva software.

***DNA fingerprinting and karyotyping.*** Analysis of the DNA fingerprinting was performed on parental blood cells and their derivative hiPS cell lines. DNA was extracted using the DNeasy Blood and Tissue Kit (Qiagen) according to the manufacturer's protocol and sent to Health Sciences Authority (HSA, Singapore) for STR analysis. Alternatively, PCR using primers specific to genomic intervals containing highly variable numbers of tandem repeats (VNTR) were used for fingerprinting analysis as described previously (Prigione et al., 2010). In brief, the PCR was performed using KAPA SYBR FAST qPCR Master Mix (2X) (KK4602) with 1 ng genomic DNA and 0.4 µM of primers in a 10 µL reaction. The PCR conditions were set as 95 °C for 3 minutes, followed by 40 cycles of 95 °C for 3 seconds and 58 °C for 30 seconds with a final extension at 72 °C for 10 minutes. The PCR products were analysed on a 2.5% agarose gel in 1 X TAE buffer with applied voltage 3 V/cm for 60 minutes. The gel with ethidium bromide stain was visualised under a UV transilluminator. For karyotyping, live hiPSC lines were sent to the Genome Institute of Singapore for analysis.

***Embryoid body formation.*** To form embryoid bodies, confluent undifferentiated hiPS cells were mechanically scraped into strips and transferred to 6-well low attachment plates in a differentiation medium consisting of DMEM supplemented with 10% fetal bovine serum (PAN Biotech), 1 X non-essential amino acids (Gibco), 1 X L-glutamine (Gibco) and, 1 X of Pen/Strep (Gibco).

***Assay for teratoma formation.*** For teratoma formation, hiPSCs grown on MEF were collected by collagenase treatment, re-suspended in Matrigel and injected into the hind limb muscle of 6-week old SCID mice. 1 well of hiPSCs in 6-well plate of 70% confluency was used for each mouse. Three mice were injected for each hiPSC clone. After 6-10 weeks, teratomas were dissected and fixed in formalin, embedded in paraffin and subjected to hematoxylin and eosin staining for all histological determinations.

***T*/*B cell rearrangement assay.*** DNA was isolated from hiPSCs and control cell lines using DNeasy Blood and Tissue Kit (Qiagen) per the manufacturer's instructions. For the PCR assay on TCR β, γ and IgH rearrangement, the primers used were described in a previous study (van Dongen et al., 2003). PCR (35 cycles of 94°C for 30 seconds, 60°C for 30 seconds, 72°C for 30 seconds) was performed using 1-2 units of Taq DNA Polymerase (Qiagen) in a 25 µL reaction with 50 ng genomic DNA, 10 pmol of each primer and 200 µM dNTP in 1 X PCR Buffer. All PCR started with initial denaturation (3 minutes at 95°C) and ended with final extension (10 minutes at 72°C) before holding at 4°C. Alternatively, the PCR was performed using KAPA SYBR FAST qPCR Master Mix (2X) (KK4602) with 1 ng genomic DNA and 0.2 µM of primers in a 10 µL reaction. The PCR and gel running conditions were same as described in DNA fingerprinting and karyotyping except that annealing temperature for PCR was set to 65 °C.

***ABO*/*Rh groups typing.*** 1 µL of finger-prick blood was mixed with 1 µL antibody solution (Anti-A, Anti-B, Anti-A,B or Anti-D, from DIAGAST) on a glass slide. The reactions were incubated for 30 seconds and the solutions were then spread and allowed to dry. The glass slides were scanned and the results were documented. The ABO and Rhesus factor genotyping was carried out as described by Muro et al., 2012 and Simsek et al., 1995, respectively. The PCR reaction and gel running conditions were same as described in DNA fingerprinting and karyotyping except that annealing temperature for PCR was set to 63.5°C and the cycle number was reduced to 35 cycles.

***ADH and ALDH genotyping.*** The primers and condition used in PCR of *ADH2, ADH3* and *ALDH2* are similar to those described by Chen et al., 1997, except that 5-10 ng of gDNA were used for each reaction and KAPA HiFi HotStart (KK2601) or KAPA SYBR FAST qPCR Master Mix (2X) (KK4602) were used for the amplifications. The PCR products were subsequently cloned into pcDNA3.3 by using a TA TOPO cloning kit (Invitrogen). At least 10 colonies were picked and sequenced.

***Cardiac differentiation.*** Human iPSCs were dissociated into single cells by incubating with Accutase (Sigma-Aldrich) at 37 °C for 5 minutes and then were seeded onto a Matrigel-coated cell culture dish at 100,000 cells/cm² in mTeSR1 medium supplemented with 5 µM ROCK inhibitor (Y-27632, Stemgent) and 10 µM CHIR99021 (Stemgent) for 24 hours. Cells were then cultured in RPMI1640 medium supplemented with B27 without insulin for two days. The medium was then changed to RPMI/B27-insulin supplemented with 5 µM IWP-2 (Stemgent) for 2 more days, and then changed to RPMI/B27-insulin for another 2 days, after which the cells were maintained in RPMI1640 medium with B27, with media change every 3 days (Lian et al., 2012). Staining for cardiomyocte markers were performed with mouse monoclonal antibodies against α-actinin (Sigma, A7811), β-myosin heavy chain (b-MHC) (Alexis, F36), and titin (Millipore, MAB1553).

***Patch clamp analysis.*** Whole cell patch-clamp recordings were performed using Axon 200B patch clamp amplifier (Axon instrument, USA) and low-pass filtered at 2-5 kHz. Data acquisition was achieved using the Digidata 1440A. Borosilicate glass electrodes (1.5 mm OD) were pulled by a horizontal puller (Model P-97, Sutter Instrument, USA) and fired-polished to a final resistance of 2-3 MΩ when filled with internal solution. The spontaneous APs were recorded from hiPSC-CMs in normal Tyrode's solution containing (in mM): NaCl 140, KCl 5.4, CaCl₂ 1.8, MgCl₂ 1, glucose 10, HEPES 10, with the pH value adjusted to 7.40 with NaOH. Solution contained (in mM): KCl 130, NaCl 5, MgCl₂ 1, MgATP 3, EGTA 10, and HEPES 10 with the pH value adjusted to 7.20 with KOH. Cells were maintained by a TC-324B single channel heater controller (Warner Instruments) at 35-37°C during the recording.

***Ethanol patch test.*** Approximately one drop of 70% ethanol was added onto a 10 × 10 mm lint pad fixed on an adhesive tape. The patch was attached to the inner surface of the upper arm for 7 minutes before removal. A patch area that shows erythema 10-15 minutes after removal is judged as positive. Controls include lint pad (without any fluid), lint pad with H₂O and lint pad with caffeine (Muramatsu et al., 1989).

***DNA microarray.*** Total RNA from PBMC, hES cells, or hiPS cells from PBMC was labeled with Cy3. Samples were hybridized to HumanHT-12v4 Beadchip (Illumina) according to manufacturer's protocol. Arrays were scanned with Beadchip Reader (Illumina). Data normalization was calculated with the Quantile normalization algorithm implemented in BioConductor lumi package. The cluster analysis was performed and scatter plots were generated in R packages. Raw data has been deposited at GEO under accession number GSE53485.

### Figure Legends

***Figure 1******. Derivation of hiPSCs from Finger-prick Blood Samples.*** (A) Schematic drawing of the strategy used in this study for reprogramming human finger-pricked blood. Images show the collection of finger-pricked blood, expansion of cells in culture and adherent colonies. The first hESC-like colony normally appeared between Day 18 to 20 and was picked after Day 21. The FPiPSCs derived can be passaged stably for long-term culture. (B) Immuno-fluorescent staining of FPiPSC colonies expressing pluripotency markers for TRA-1-60, SSEA4 and OCT4. Hoechst staining indicates the total cell content per field. (C) Bisulfite DNA methylation analysis on the promoter of *OCT4* and *NANOG* in FPiPSCs and in their parental cells. The white and black circles represent unmethylated and methylated CpG islands in the promoter region, respectively. (D) Dendrogram showing un-supervised hierarchical clustering of the global expression profiles of hESCs, hiPSCs and parental blood cells (Top). Heat-map analysis of the hESCs, hiPSCs and parental blood cells for expression of Pluripotency and Hematopoietic specific marker genes (Bottom). Red represents up-regulation, black represents down-regulation. (E) Scatter plots comparing FPiPSCs global gene expression profiles to parental cells (left) and H7 human ESCs (right). The black lines indicate the linear equivalent and 2-fold changes in gene expression levels between the paired cell types. Positions of pluripotency genes *OCT4, SOX2, NANOG,* and *LIN28* in scatter plots are indicated. (F) qRT-PCR analyses for the expression of ESC marker genes *OCT4, SOX2, NANOG, LIN28, DNMT3B, REX1*, *KLF4* and *c-MYC* in FPiPSCs, H7 ESCs and parental cells. Individual PCR reactions were normalized to H7 ESC and *β-ACTIN* as internal control. All the FPiPSC lines examined showed similar expression of ESC marker genes to that of H7 ESCs.

***Figure 2******. FPiPSCs Differentiate into Derivatives of Three Germ Layers.*** (A) Immuno-fluorescent staining of differentiation markers after *in vitro* differentiation of FPiPSCs. Ectoderm was stained by GFAP and β3-tubulin antibodies whereas endoderm was stained by antibody against GATA4. All images in the upper panel were acquired with 10x objectives except for GATA4 staining image which was acquired with 4x objectives. Lower panel images are shown in higher magnification. Hoechst staining indicates the nucleus. (B) Immuno-fluorescent staining of α-actinin, β-myosin heavy chain (β-MHC) and titin in hiPSCs derived cardiomyocyctes. Hoechst staining indicates the nucleus. (C) Patch clamp analyses of hiPSCs derived cardiomyocyctes. (D) Hematoxylin and eosin staining of teratomas derived from immune-deficient mice injected with FPiPSCs show tissues representing all three embryonic germ layers including gut like epithelium (endoderm), cartilage (mesoderm) and neuro tissue (ectoderm).

***Figure 3******. Characterizations of the FPiPSCs.*** (A) Transgene expression analysis of the FPiPSC lines derived. The SeV (Sendai virus) backbone and SeV *OCT4* expression values were measured by qRT-PCR and normalized to SeV infected parental cell with *β-ACTIN* as internal control. No SeV transgene was detected in the total RNA harvested from these hiPSC lines. (B) Cytogenetic analysis on FPiPSCs. The FPiPSCs showed normal human karyotype with 46 chromosomes. (C) IgH rearrangement analysis on the FPiPSCs clones. LTR-228 (B-cell line) was used as a positive control. No IgH recombination was observed in our FPiPSCs. (D) TCRB rearrangement analysis on the FPiPSCs clones. Jurkat (T-cell line) or CD3+ T-cells were used as positive controls for this analysis. (E) TCRG rearrangement analysis on the FPiPSCs clones. Jurkat cells (T-cell line) or CD3+ T-cells were used as positive controls for this analysis. Negative results from all the V(D)J rearrangement PCR implies that the FPiPSCs derived did not originate from B or T lymphocytes. In all figures in (C), (D) and (E), the expected PCR product range was stated on the left and the target regions were indicated below the gel images. The FP blood used in this assay had been cultured in cell expansion medium for at least 15 days before harvested. A water sample was always included in the PCR analysis as a negative control. (F) The genomic DNA amplification control experiment was performed on all the samples used in these V(D)J rearrangement analyses.

***Figure 4******. DIY concept for FPiPSCs generation.*** (A) FP blood samples collected 12 hours, 24 hours and 48 hours prior to cell culture. The cells were RBC-lysed at day 0 (left) and expanded for 12 days in medium. All images were acquired with 20x objectives. (B) hiPSC colonies derived from Donor 1 DIY FP blood (12 hours and 24 hours) stained positive for a pluripotency marker, TRA-1-60. The upper image was acquired with 20x objectives whereas the lower image was acquired with 4x objectives. (C) hiPSC colonies derived from Donor 5 DIY FP blood (24 hours and 48 hours) stained positive for a pluripotency marker, TRA-1-60. All image were acquired with 10x objectives. (D) qRT-PCR analyses for the expression of ESC marker genes *OCT4, SOX2, NANOG, LIN28, DNMT3B, REX1*, and *c-MYC* in FP(DIY)iPSCs, H1 ESCs and parental cells. Individual PCR reactions were normalized to H1 ESC and *β-ACTIN* as internal control. All the FP(DIY)iPSC lines examined showed similar expression of ESC marker genes to that of H1 ESCs. (E) DNA fingerprinting analysis confirms that FPiPS cell lines are derived from their parental lines. PCR primer sets D10S1214, D17S1290, D7S796, and D21S2055 were used to detect for variable tandem repeats. (F) Summary of hiPSCs derived from donors. The VP and FP indicated were the venipuncture blood and finger-pricked blood respectively. The reprogramming yield was calculated based on the TRA-1-60 colonies observed and normalized to 1 mL of blood.

***Figure 5******. Blood typing and DNA genotype the finger-prick samples.*** (A) Blood grouping of the donors from finger-prick samples. The upper panel show the antibodies used. If the erythrocytes of the donor carry the corresponding antigen, agglutination will take place and the donors' blood groups can be determined. The phenotypic blood group for Donor 1 and Donor 2 were Type B+, while Donor 5 was O-. (B) Genotyping of the donors' blood group from finger-prick blood and derived hiPSCs. Specific primers were used for *ABO* alleles detection. The upper panel shows the specific allele that the primers amplified. The primer 6O, 6AB, 7AO and 7B were designed to target the O allele exon 6, A or *B* allele exon 6, A or O allele exon 7 and *B* allele exon 7 respectively. A clear DNA band (-100 bp) indicates a positive PCR result and shows that the donor carries these alleles. Donor 1 FP blood and FPiPSC shows the BB genotype (upper panel) whereas Donor 2 FP blood and FPiPSC shows the BO genotype (lower panel). The primers targeting the reference gene *36B4* was included as a positive control for genomic DNA amplification. (C) Rhesus D antigen genotyping of the finger-prick samples and derived hiPSCs. The primers were designed to target both *Rh CcEe* and *Rh D* region. The 1200 bp band is amplified from the *Rh CcEe* alleles and 600 bp is from *Rh D* allele. For Rh D positive samples, a clear 600 bp band and a 1200 bp band can be observed; otherwise, only 1200 bp band can be detected. Donor 1 and 2 and their hiPSCs showed a Rh D positive genotype. (D) Genotyping of the donors' blood group from DIY finger-prick blood derived hiPSCs. Donor 5 FP(DIY)iPSCs showed a OO genotype. (E) Rhesus D antigen genotyping of the DIY finger-prick samples derived hiPSCs. Donor 1 FP(DIY)iPSCs showed Rh D positive genotype while Donor 5 FP(DIY)iPSCs is Rh D negative. (F) SNP on the *ALDH2* loci. *ALDH2* is responsible for conversion of acetaldehyde to acetic acid. *ALDH2***2* allele consists of the glutamate to lysine mutation (1510 G →A) and encodes a defective *ALDH2.* (G) Ethanol patch test on the donors. Donor 1 showed a flush reaction to ethanol whereas Donor 2 and 5 showed no effect to ethanol when it was applied onto the donor's skin. (H) The upper panel shows the metabolic pathway for degradation of ethanol in the body. The ethanol is first oxidized into toxic acetaldehyde by alcohol dehydrogenase and subsequently oxidized to acetic acid by ALDH. In our study, Donor 1 possesses an *ALDH2***2* allele and thus manifested an intolerance to ethanol.

***Figure 6******. Derivation of Venipuncture (VP) Blood hiPSCs.*** (A) Schematic drawing of the timeline for VP blood reprogramming. The small volume of VP blood (250 µl and 500 µl) was collected and cells were expanded for 12 days. The colonies were observed after 18 days of SeV transduction. The VPiPSCs derived were stable for long term culture. (B) Immuno-fluorescent staining of pluripotency markers on VPiPSCs. All the VPiPSCs stained positive for OCT4, TRA-1-60 and SSEA4. The corresponding Hoechst 33342 staining indicates the total cell content in the field. (C) Pluripotency gene expression analysis on VPiPSCs. The pluripotency genes examined are listed on the upper panel of the figure. Individual PCR reactions were normalized to H7 ESC and *β-ACTIN* as internal controls. All the FPiPSC lines examined showed a similar expression of ESC marker genes as H7 ESC. (D) Transgene expression analysis of the VPiPSC lines after 4 passages. The SeV backbone expression values were measured by qRT-PCR and normalized to SeV-infected parental cells with *β-ACTIN* as internal control. No SeV backbone was detected in the total RNA harvested from these iPSC lines.

***Figure 7******. Characterization of VPiPSCs.*** (A) DNA methylation analysis on the promoter of *OCT4* and *NANOG* in the VPiPSCs. The white and black circles represent unmethylated and methylated CpG islands in the promoter region, respectively. (B) Immuno-fluorescent staining of mesoderm markers, Desmin and SMA, of *in vitro* differentiated VPiPSCs. (C) Immuno-fluorescent staining of differentiation markers after *in vitro* differentiation of VPiPSCs. Ectoderm was stained by GFAP antibodies.

***Figure 8******. Isolation and Expansion of FP Blood Cells.*** (A) Collection of FP blood. The figures are shown in a sequential manner (I to IV). The finger-prick was applied on the donor's finger tip (I) and the blood was subsequently collected into a Microtainer (II to IV). (B) RBC depletion of FP blood. 10 µl of FP blood were lysed in RBC lysis buffer (from I to IV). (C) Images of the purified FP blood cells from 5-12 days. All images were acquired with 20x objectives. (D) Cells were analyzed by flow cytometry to determine the cell-surface immunophenotype on specific days of culture (Day 0, 8, 15). Percentage of cells with cell-surface immunophenotypes, CD3(+), CD19(+), CD34 (+) or CD71(+) are summarized and represented in the table. Except for CD34 sample, dead cells were excluded by propidium iodide staining.

***Figure 9******. V(D)J Rearrangement of VPiPSCs Derived.*** (A), (B) and (C) show the IgH, TCRB and TCRG rearrangement analysis on VPiPSCs derived. LTR-288 was used as the positive control for the IgH rearrangement analysis whereas Jurkat and CD3+ T-cells were used as positive controls for both the TCRB and TCRG rearrangement analysis. Negative results from all the V(D)J rearrangement PCR implies that the VPiPSCs derived did not originate from B or T lymphocytes. (D) Amplification control on VPiPSCs genomic DNA which was used in the analysis. All the samples show 5 bands which indicates that the genomic DNA used was of good quality. In all figures, the expected PCR product ranges are stated on the left and the target regions are indicated below the gel images. Also, a water sample is always included in the PCR test as a negative control.

***Figure 10******. V(D)J Rearrangement of FP(DIY)iPSCs Derived.*** (A), (B) and (C) show the IgH, TCRB and TCRG rearrangement analysis on FP(DIY)iPSCs derived. Results implies that the FP(DIY)iPSCs derived did not originate from B or T lymphocytes. (D) Amplification control on FP(DIY)iPSCs genomic DNA which was used in the analysis. All the samples show 5 bands which indicates that the genomic DNA used was of good quality.

***Figure 11******. Blood typing and DNA genotyping of DIY Finger-prick blood and derivative iPSCs**.* (A) Genotyping of the Donor 5 blood group from DIY finger-prick samples. Result shows the expected OO genotype. (B) Rhesus D antigen genotyping of Donor 5 DIY finger-prick samples. Result shows the expected Rhesus negative genotype. (C) Blood grouping of Donor 1 DIY finger-prick samples obtained 24 hours and 48 hours prior to analysis. Donor 1 shows the expected B+ phenotype. (D) Genotyping of the donors' blood group from DIY finger-prick blood derived hiPSCs. Donor 1 FP(DIY)iPSC shows the expected BB genotype.

***Figure 12******. Genotyping of ALDH2, ADH2 and ADH3 on hiPSCs derived.*** (A) and (B) showed the sequencing alignment results on *ALDH2* of Donor 1 and Donor 2 FP blood (A) and their respective hiPSCs (B). Both the normal and the mutant *ALDH2* allele were found in Donor 1's sequencing results indicating that Donor 1 is heterozygous for *ALDH2.* Donor 2 on the other hand, carries only the normal allele. (C) Ethanol patch test on Donor 1. Caffeine, 70% ethanol, water and a blank patch were applied on Donor 1's skin (0 min). After 10 minutes, the region which was applied with 70% ethanol showed flush reaction as indicated by the white arrow. (D) Summary of genotyping *ADH2, ADH3* and *ALDH2* on FPiPSCs. Donor 1's hiPSCs show heterozygosity for all the 3 genes whereas Donor 2 and 3 hiPSCs show homozygote mutant alleles of *ADH2* but homozygote normal alleles on both *ADH3* and *ALDH2.* Donor 5 hiPSCs show heterozygosity for *ADH3* but normal alleles for *ADH2* and *ALDH2.*

***Figure 13******. Contains Table 1: Fingerprint analysis data of iPSC clones.***

***Figure 14******. Contains Table 2: Summary of Characterizations performed on iPSC clones.***

***Figure 15******. Schematic depiction of an integrative strategy for hiPSCs banking.*** Illustration of the integrative strategy for hiPSCs banking. Using FP blood reprogramming, hiPSC facilities can recruit a diverse cohort of donors world-wide. hiPSC facility provides a kit containing sterile finger-prick and blood container to the donor. The donors will complete the informed consent form and the health questionnaire and return them back to the facility together with their FP blood. The facility is able to do a series of DNA sequencing, serological assays as well as hiPSC derivation from a single drop of FP blood sample.

### Results

***Generation of hiPSCs from sub-milliliter of human peripheral blood.*** Using 2 mL of human peripheral blood mononuclear cells (PBMCs), T-cells and non T-cells were successfully reprogrammed from two individuals (data not shown). This is in good agreement with previous studies which start with similar volumes of blood samples for reprogramming (Merling et al., 2013; Ye et al., 2013).

It was examined whether the same Ficoll purification protocol would yield enough cells from blood samples less than 1 mL in volume. The starting volume of blood was gradually reduced, and it was confirmed that reprogramming can be achieved with as little as 250 µL to 500 µL of venous blood (Figure 6A). Using immuno-fluorescence, venipuncture derived iPSCs (VPiPSCs) stained positive for OCT4, TRA-1-60 and SSEA-4 (Figure 6B). qRT-PCR results showed the re-activation of pluripotency markers (Figure 6C). Notably, the Sendai virus transgenes were not detected in the VPiPSCs after 4 passages in culture (Figure 6D). Consistent with their hESC-like morphology, VPiPSCs show demethylation of CpG dinucleotides at the *OCT4* and *NANOG* promoters (Figure 7A), and are able to differentiate into different lineages *in vitro* (Figure 7B and 7C). DNA fingerprinting analysis verified that these cells were indeed derived from the parental blood cells (Table 1 in Figure 13).

However, when this approach of venipuncture followed by Ficoll-Paque centrifugation was used on samples for which the starting blood volume was below 250 µL, sufficient PBMCs for reprogramming were not obtained. One possibility for this result could be technical difficulties associated with isolating the buffy coat, resulting in loss of mononuclear cells.

***Derivation of hiPSCs from finger-prick blood samples.*** To investigate if hiPSCs could be generated from minute amounts of blood samples, the purification process was changed to exclude the use of conventional Ficoll purification. Finger-prick samples were collected from different individuals (Figure 8A) and subjected the cells to hypotonic lysis solution for depletion of the red blood cells (Figure 8B). Strikingly, it was found that the RBC-lysed finger-prick cells from 10 µL of blood can be expanded in medium culture to yield enough dividing cells for reprogramming (Figure 8C).

In total, 5 donors (19 to 36 years old) were recruited, out of which 4 donors resulted in successful reprogramming to yield hiPSCs. No noticeable reduction in reprogramming efficiency was observed as a function of donors' age. The one donor (aged 29 year old) did not give rise to enough cells in culture, and thus viral infection for reprogramming was not performed on samples from this donor.

Consistent with previous reports (Chou et al., 2011; Yang et al, 2012), the cells after 15 days of culture expansion were largely CD71+ myeloid progenitors (Figure 8D). Using this approach, the appearance of hESC-like colonies were observed 20 days after transduction with Sendai virus (Figure 1A). All finger-prick derived iPSC (FPiPSC) clones were propagated for at least 20 passages and remained stable in culture.

The expression of pluripotency markers in the FPiPSC lines was examined using immuno-staining. In line with the hESC-like morphology, expression of OCT4, TRA-1-60 and SSEA-4 was detected (Figure 1B). Consistent with the activation of pluripotency-associated genes, reprogramming of the finger-prick blood cells was accompanied by the demethylation of CpG dinucleotides at the *OCT4* and *NANOG* promoters (Figure 1C).

Global gene expression analysis of the FPiPSCs was performed, with comparison to hESCs and somatic parental cells. Clustering dendrogram revealed a high degree of similarity among the reprogrammed hiPSCs which clustered together with the H1, H7 and H9 ESCs and were distant from the parental somatic cells (Figure 1D, Top). Heat-map analysis indicates the re-activation of pluripotency genes and the suppression of hematopoietic genes in the hiPSC lines (Figure 1D). Analysis of scatter plots similarly shows a tighter correlation between reprogrammed FPiPSCs and human ESCs (H7 ESCs) rather than with the parental blood cells (Figure IE). Quantitative PCR analysis confirmed the expression of endogenous pluripotency markers including *OCT4, SOX2, KLF4, c-MYC, REX1*, *NANOG, LIN28* and *DNMT3B* (Figure 1F). Notably, these genes were restored in the FPiPSCs to levels similar to that observed in H7 hESCs (Figure 1F).

***FPiPSCs differentiate into derivatives of three germ layers.*** The developmental potential of the FPiPSC lines was evaluated by *in vitro* embryoid body (EB) differentiation. The hiPSCs readily formed EBs upon induction and are capable of differentiating into mesodermal (Desmin and SMA), neural (β3-Tubulin, GFAP) and endodermal (GATA4) lineages (Figure 2A).

Following specific *in vitro* differentiation, FiPSCs gave rise to rhythmically beating cardiomyocytes. Positive staining for α-actinin, β-MHC and titin confirmed their cardiomyocyte identities (Figure 2B). Using patch clamp analysis, individual cardiomyocytes were characterized and found to have developed distinct ventricular, atrial, and nodal action potentials (Figure 2C).

The most rigorous test for the pluripotency of human iPSCs is the formation of teratomas in immune-deficient mouse hosts (Lensch et al., 2007). Upon injection into the hind limb muscle of SCID mice, the FPiPSC lines generated well-differentiated cystic teratomas comprising structures and tissues derived from the three embryonic germ layers. These included gut-like epithelium, cartilage, and neuro-tissues (Figure 2D).

***FPiPSCs are free from transgenes and V(D)J rearrangement.*** Efficient transgene silencing is essential for the derivation of pluripotent hiPSC lines (Park et al., 2008). qRT-PCR using primers specific for the Sendai vector backbone and the exogenous Sendai *OCT4* reprogramming factor confirmed that the FPiPSCs do not carry any Sendai transgenes (Figure 3A). The FPiPSC lines examined exhibited normal karyotypes (Figure 3B). The DNA fingerprinting analysis verified that these cells were indeed derived from the parental blood cells and were not a result of contamination from existing hESC or hiPSC lines (Table 1 in Figure 13).

B-cells and T-cells are abundant in circulating blood and the latter can easily be reprogrammed (Loh et al., 2010; Seki et al., 2010; Staerk et al., 2010). The FPiPSC clones were therefore tested for TCR and IgH rearrangement using PCR-based detection. While the clonal controls (Jurkat and LTR-228) and CD3+ T-cells show bands indicative of rearrangement, all the FPiPSC and VPiPSC clones examined did not have any rearrangement at the TCR β, γ and IgH loci (Figure 3C, 3D, 3E, 3F and 9). Thus, these results confirm that the FPiPSCs did not originate from lymphoid T and B cells.

***Reprogramming from DIY finger prick samples.*** Unlike venipuncture which can only be performed by trained phlebotomists, finger-prick blood can be collected by the donors themselves with proper instruction. To test this idea, donors were directed to prick their own fingers in a normal room environment and collect a single drop of blood sample into an EDTA tube. The tube was placed on ice and delivered to the lab for reprogramming. 12, 24 or 48-hours later, the cells were treated with RBC lysis buffer and observed under the microscope for viability and signs of contamination (Figure 4A). Interestingly, after 12 days of expansion in medium, the cells appeared healthy and were actively dividing (Figure 4A).

To commence reprogramming, cells isolated from the finger-prick samples were transduced with Sendai virus. hESC-like colonies were observed about 20 days post-infection. In total, 8 TRA-1-60+ colonies were obtained from two donors in four independent experiments (Figure 4B).

V(D)J re-arrangement PCR confirmed that the FP(DIY)iPSCs were not originated from lymphoid T and B cells (Figure 10). Quantitative PCR analysis indicates the expression of endogenous pluripotency marker genes to the level comparable with H1 ESCs (Figure 4D). Notably, the FP(DIY)iPSCs were derived from respective donor FP cells and not due to contamination from existing hESCs or hiPSCs (Figure 4E). The efficiency of 100-300 colonies per mL is similar to the reprogramming results from fresh samples taken under a controlled and clean laboratory setting. Interestingly, no noticeable reduction in reprogramming efficiency was observed between freshly collected and DIY finger-prick samples (Figure 4F).

In summary, transgene-free and V(D)J re-arrangement free hiPSCs were successfully derived from sub-milliliters volumes of venipuncture and single drop volumes of finger-prick samples (Figure 4F and Table 2 in Figure 14), with a high reprogramming yield of 100-600 colonies per mL of blood (Figure 4F).

***Integrative strategy for hiPSC banking.*** Since a single drop of blood is approximately 20 µL and only 10 µL is required for reprogramming, it was then assessed whether the remaining amount of the donors' blood could be used for other analyses. Pluripotent stem cells can potentially be an unlimited resource for cell therapy, but immune rejection may pose problems during transplantation. Apart from the Human Leukocyte Antigen (HLA) (Taylor et al., 2012), a recent study discovered that *in vitro* hESCs differentiated hepatocytes and cardiomyocytes express ABO antigens (Molne et al., 2008).

Using 4 µL of finger-prick blood, a serological analysis of A, B and Rh antigen groups was performed for three donors (Figure 5A). gDNA was then harvested from finger-prick samples; 2 µL of blood yielded approximately 150 ng of PCR-grade gDNA for molecular studies. ABO/Rh typing can also be determined using PCR based technology. Moreover, PCR analysis can decipher the exact genotype of the ABO groups. The results demonstrate that although both Donor 1 and 2 have a phenotype of B+, Donor 1 has a genotype of BB+ while Donor 2 is BO+ (Figure 5B, 5C). On the other hand, Donor 5 is recessive for both the ABO and Rh antigens as shown by the O- phenotype (Figure 5A). Genotypic analysis of Donor 5 finger-prick blood corroborated with the serological results (Figure 11A, 11B). Interestingly, DIY finger-prick blood samples show no sign of deterioration (24 and 48-hour after sample collection) as the donors' serological ABO/Rh blood type could still be accurately determined (Figure 11C). Expectedly, both the FPiPSCs and FP(DIY)iPSCs derived from the respective donors exhibited the same ABO and Rh genotype as the parental blood cells (Figure 5B, 5C, 5D, 5E and 11D).

Expansion of the DIY finger-prick samples collected 72 hours prior to cell culture, a high degree of cell death was observed.

To further evaluate if the gDNA isolated from the small amount of finger-prick blood samples can be used to detect single nucleotide mutations, sequencing analysis was performed on the aldehyde dehydrogenase (*ALDH*) and alcohol dehydrogenase (*ADH*) genes involved in alcohol metabolism. Interestingly, the sequencing results on *ALDH2* indicate that Donor 1 carries a mutant *ALDH2*2* allele (1510 G>A mutation) (Figure 4F). The mutation was observed in both the blood sample and the derivative hiPSCs (Figure 12A and 12B). Various reports have suggested that the single nucleotide polymorphism (SNP) on *ALDH2* could lead to protein malfunction resulting in the accumulation of toxic acetaldehyde in the body, and manifests as a red flush on the skin (Rao et al., 2007; Xiao et al, 1995).

Donors were next asked to perform a simple test when the finger-prick samples were collected. Consistent with the *ALDH2* genotype, an ethanol patch test performed by Donor 1 resulted in redness of the skin, whereas Donor 2 and 5 who carried the homozygous normal *ALDH2***1* alleles did not display any flush syndrome (Figure 5G and Figure 12C).

In summary, using a combination of DNA sequencing and a DIY ethanol test, the level of alcohol tolerance for Donor 1, 2 and 5 (Figure 5H and 12D) were profiled. Importantly, the hiPSCs generated faithfully recapitulate the mutant genotypes and will be a good resource for future study of alcohol metabolism (Figure 12D).

A model of a system for finger prick blood collection by donors and integration into an analysis and hiPSC bank is shown in Figure 15.

The citation of any publication is for its disclosure prior to the filing date and should not be construed as an admission that the present invention is not entitled to antedate such publication by virtue of prior invention.

As used in this specification and the appended claims, the singular forms "a", "an" and "the" include plural reference unless the context clearly dictates otherwise.

As used in this specification and the appended claims, the terms "comprise", "comprising", "comprises" and other forms of these terms are intended in the non-limiting inclusive sense, that is, to include particular recited elements or components without excluding any other element or component.

As used in this specification and the appended claims, all ranges or lists as given are intended to convey any intermediate value or range or any sublist contained therein.

Unless defined otherwise all technical and scientific terms used herein have the same meaning as commonly understood to one of ordinary skill in the art to which this invention belongs.

### References

Takahashi, K., Tanabe, K., Ohnuki, M., Narita, M., Ichisaka, T., Tomoda, K., and Yamanaka, S. (2007). Induction of Pluripotent Stem Cells from Adult Human Fibroblasts by Defined Factors. Cell 131, 861-872.
Yu, J., Vodyanik, M.A., Smuga-Otto, K., Antosiewicz-Bourget, J., Frane, J.L., Tian, S., Nie, J., Jonsdottir, G.A., Ruotti, V., Stewart, R., et al. (2007). Induced pluripotent stem cell lines derived from human somatic cells. Science 318, 1917-1920.
Park, I.-H., Zhao, R., West, J.A., Yabuuchi, A., Huo, H., Ince, T.A., Lerou, P.H., Lensch, M.W., and Daley, G.Q. (2008). Reprogramming of human somatic cells to pluripotency with defined factors. Nature 451, 141-146.
Kondo, T., Asai, M., Tsukita, K., Kutoku, Y., Ohsawa, Y., Sunada, Y., Imamura, K., Egawa, N., Yahata, N., Okita, K., et al. (2013). Modeling Alzheimer's Disease with iPSCs Reveals Stress Phenotypes Associated with Intracellular Aβ and Differential Drug Responsiveness. Cell Stem Cell 12, 487-496.
Park, I.H., Arora, N., Huo, H., Maherali, N., Ahfeldt, T., Shimamura, A., Lensch, M.W., Cowan, C., Hochedlinger, K., and Daley, G.Q. (2008). Disease-specific induced pluripotent stem cells. Cell 134, 877-886.
Loh, Y.H., Agarwal, S., Park, I.H., Urbach, A., Huo, H., Heffner, G.C., Kim, K., Miller, J.D., Ng, K., and Daley, G.Q. (2009). Generation of induced pluripotent stem cells from human blood. Blood 113, 5476-5479.
Ye, Z., Zhan, H., Mali, P., Dowey, S., Williams, D.M., Jang, Y.Y., Dang, C.V., Spivak, J.L., Moliterno, A.R., and Cheng, L. (2009). Human-induced pluripotent stem cells from blood cells of healthy donors and patients with acquired blood disorders. Blood 114, 5473-5480.
Giorgetti, A., Montserrat, N., Aasen, T., Gonzalez, F., Rodriguez-Piza, I., Vassena, R., Raya, A., Boue, S., Barrero, M.J., Corbella, B.A., et al. (2009). Generation of induced pluripotent stem cells from human cord blood using OCT4 and SOX2. Cell Stem Cell 5, 353-357.
Haase, A., Olmer, R., Schwanke, K., Wunderlich, S., Merkert, S., Hess, C., Zweigerdt, R., Gruh, I., Meyer, J., Wagner, S., et al. (2009). Generation of induced pluripotent stem cells from human cord blood. Cell Stem Cell 5, 434-441.
Sohn, S.K., Kim, J.G., Seo, K.W., Chae, Y.S., Jung, J.T., Suh, J.S., and Lee, K.B. (2002). GM-CSF-based mobilization effect in normal healthy donors for allogeneic peripheral blood stem cell transplantation. Bone Marrow Transplant 30, 81-86.
Loh, Y.H., Hartung, O., Li, H., Guo, C., Sahalie, J.M., Manos, P.D., Urbach, A., Heffner, G.C., Grskovic, M., Vigneault, F., et al. (2010). Reprogramming of T cells from human peripheral blood. Cell Stem Cell 7, 15-19.
Seki, T., Yuasa, S., Oda, M., Egashira, T., Yae, K., Kusumoto, D., Nakata, H., Tohyama, S., Hashimoto, H., Kodaira, M., et al. (2010). Generation of induced pluripotent stem cells from human terminally differentiated circulating T cells. Cell Stem Cell 7, 11-14.
Staerk, J., Dawlaty, M.M., Gao, Q., Maetzel, D., Hanna, J., Sommer, C.A., Mostoslavsky, G., and Jaenisch, R. (2010). Reprogramming of human peripheral blood cells to induced pluripotent stem cells. Cell Stem Cell 7, 20-24.
Serwold, T., Hochedlinger, K., Inlay, M.A., Jaenisch, R., and Weissman, I.L. (2007). Early TCR expression and aberrant T cell development in mice with endogenous prerearranged T cell receptor genes. J Immunol 179, 928-938.
Chou, B.K., Mali, P., Huang, X., Ye, Z., Dowey, S.N., Resar, L.M., Zou, C., Zhang, Y.A., Tong, J., and Cheng, L. (2011). Efficient human iPS cell derivation by a non-integrating plasmid from blood cells with unique epigenetic and gene expression signatures. Cell Res 21, 518-529.
Mack, A.A., Kroboth, S., Rajesh, D., and Wang, W.B. (2011). Generation of induced pluripotent stem cells from CD34+ cells across blood drawn from multiple donors with non-integrating episomal vectors. PLoS One 6, e27956.
Okita, K., Yamakawa, T., Matsumura, Y., Sato, Y., Amano, N., Watanabe, A., Goshima, N., and Yamanaka, S. (2013). An efficient nonviral method to generate integration-free human-induced pluripotent stem cells from cord blood and peripheral blood cells. Stem Cells 31, 458-466.
Merling, R.K., Sweeney, C.L., Choi, U., De Ravin, S.S., Myers, T.G., Otaizo-Carrasquero, F., Pan, J., Linton, G., Chen, L., Koontz, S., et al. (2013). Transgene-free iPSCs generated from small volume peripheral blood nonmobilized CD34+ cells. Blood 121, e98-107.
Ye, L., Muench, M.O., Fusaki, N., Beyer, A.I., Wang, J., Qi, Z., Yu, J., and Kan, Y.W. (2013). Blood cell-derived induced pluripotent stem cells free of reprogramming factors generated by Sendai viral vectors. Stem Cells Translational Medicine 2, 558-566.
van den Akker, E., Satchwell, T.J., Pellegrin, S., Daniels, G., and Toye, A.M. (2010). The majority of the in vitro erythroid expansion potential resides in CD34(-) cells, outweighing the contribution of CD34(+) cells and significantly increasing the erythroblast yield from peripheral blood samples. Haematologica 95, 1594-1598.
Yang, W., Mills, J.A., Sullivan, S., Liu, Y., French, D.L., and Gadue, P. (2012). iPSC Reprogramming from human peripheral blood using sendai virus mediated gene transfer StemBook.
Freberg, C.T., Dahl, J.A., Timoskainen, S., and Collas, P. (2007). Epigenetic reprogramming of OCT4 and NANOG regulatory regions by embryonal carcinoma cell extract. Mol Biol Cell 18, 1543-1553.
Prigione, A., Fauler, B., Lurz, R., Lehrach, H., and Adjaye, J. (2010). The Senescence-Related Mitochondrial/Oxidative Stress Pathway is Repressed in Human Induced Pluripotent Stem Cells. Stem Cells 28, 721-733.
van Dongen, J.J., Langerak, A.W., Bruggemann, M., Evans, P.A., Hummel, M., Lavender, F.L., Delabesse, E., Davi, F., Schuuring, E., Garcia-Sanz, R., et al. (2003). Design and standardization of PCR primers and protocols for detection of clonal immunoglobulin and T-cell receptor gene recombinations in suspect lymphoproliferations: report of the BIOMED-2 Concerted Action BMH4-CT98-3936. Leukemia 17, 2257-2317.
Muro, T., Fujihara, J., Imamura, S., Nakamura, H., Kimura-Kataoka, K., Toga, T., Iida, R., Yasuda, T., and Takeshita, H. (2012). Determination of ABO genotypes by real-time PCR using allele-specific primers. Leg Med (Tokyo) 14, 47-50.
Simsek, S., Faas, B.H., Bleeker, P.M., Overbeeke, M.A., Cuijpers, H.T., van der Schoot, C.E., and von dem Borne, A.E. (1995). Rapid Rh D genotyping by polymerase chain reaction-based amplification of DNA. Blood 85, 2975-2980.
Chen, W.J., Loh, E.W., Hsu, Y.P., and Cheng, A.T. (1997). Alcohol dehydrogenase and aldehyde dehydrogenase genotypes and alcoholism among Taiwanese aborigines. Biol Psychiatry 41, 703-709.
Lian, X., Hsiao, C., Wilson, G., Zhu, K., Hazeltine, L.B., Azarin, S.M., Raval, K.K., Zhang, J., Kamp, T.J., and Palecek, S.P. (2012). Cozzarelli Prize Winner: Robust cardiomyocyte differentiation from human pluripotent stem cells via temporal modulation of canonical Wnt signaling. Proceedings of the National Academy of Sciences 109, E1848-E1857.
Muramatsu, T., Higuchi, S., Shigemori, K., Saito, M., Sasao, M., Harada, S., Shigeta, Y., Yamada, K., Muraoka, H., Takagi, S., et al. (1989). Ethanol patch test--a simple and sensitive method for identifying ALDH phenotype. Alcohol Clin Exp Res 13, 229-231.
Lensch, M.W., Schlaeger, T.M., Zon, L.I., and Daley, G.Q. (2007). Teratoma formation assays with human embryonic stem cells: a rationale for one type of human-animal chimera. Cell Stem Cell 1, 253-258.
Taylor, C.J., Peacock, S., Chaudhry, A.N., Bradley, J.A., and Bolton, E.M. (2012). Generating an iPSC bank for HLA-matched tissue transplantation based on known donor and recipient HLA types. Cell Stem Cell 11, 147-152.
Molne, J., Bjorquist, P., Andersson, K., Diswall, M., Jeppsson, A., Strokan, V., Rydberg, L., and Breimer, M.E. (2008). Blood group ABO antigen expression in human embryonic stem cells and in differentiated hepatocyte- and cardiomyocyte-like cells. Transplantation 86, 1407-1413.
Rao, V.R., Bhaskar, L.V., Annapurna, C., Reddy, A.G., Thangaraj, K., Rao, A.P., and Singh, L. (2007). Single nucleotide polymorphisms in alcohol dehydrogenase genes among some Indian populations. Am J Hum Biol 19, 338-344.
Xiao, Q., Weiner, H., Johnston, T., and Crabb, D.W. (1995). The aldehyde dehydrogenase ALDH2*2 allele exhibits dominance over ALDH2*1 in transduced HeLa cells. J Clin Invest 96, 2180-2186.
Rao, M. (2013). Public Private Partnerships: A Marriage of Necessity. Cell Stem Cell 12, 149-151.
Turner, M., Leslie, S., Martin, Nicholas G., Peschanski, M., Rao, M., Taylor, Craig J., Trounson, A., Turner, D., Yamanaka, S., and Wilmut, I. (2013). Toward the Development of a Global Induced Pluripotent Stem Cell Library. Cell Stem Cell 13, 382-384.
Fusaki, N., Ban, H., Nishiyama, A., Saeki, K., and Hasegawa, M. (2009). Efficient induction of transgene-free human pluripotent stem cells using a vector based on Sendai virus, an RNA virus that does not integrate into the host genome. Proc Jpn Acad Ser B Phys Biol Sci 85, 348-362.
Chen, G., Gulbranson, D.R., Hou, Z., Bolin, J.M., Ruotti, V., Probasco, M.D., Smuga-Otto, K., Howden, S.E., Diol, N.R., Propson, N.E., et al. (2011). Chemically defined conditions for human iPSC derivation and culture. Nature Methods 8, 424-429.
Zhou, T., Benda, C., Duzinger, S., Huang, Y., Li, X., Li, Y., Guo, X., Cao, G., Chen, S., Hao, L., et al. (2011). Generation of induced pluripotent stem cells from urine. J Am Soc Nephrol 22, 1221-1228.
Xue, Y., Cai, X., Wang, L., Liao, B., Zhang, H., Shan, Y., Chen, Q., Zhou, T., Li, X., Hou, J., et al. (2013). Generating a non-integrating human induced pluripotent stem cell bank from urine-derived cells. PLoS ONE 8, e70573.
Cong, L., Ran, F.A., Cox, D., Lin, S., Barretto, R., Habib, N., Hsu, P.D., Wu, X., Jiang, W., Marraffini, L.A., et al. (2013). Multiplex Genome Engineering Using CRISPR/Cas Systems. Science 339, 819-823.
Geti, I., Ormiston, M.L., Rouhani, F., Toshner, M., Movassagh, M., Nichols, J., Mansfield, W., Southwood, M., Bradley, A., Rana, A.A., et al. (2012). A practical and efficient cellular substrate for the generation of induced pluripotent stem cells from adults: blood-derived endothelial progenitor cells. Stem Cells Transl Med 1, 855-865.

## Claims

1. A method of inducing pluripotency in a hematopoietic cell, the method comprising:
providing a blood sample, wherein the volume of the blood sample is from 10 µl to 250 µl of blood collected from a subject in the absence of any polysaccharide preparation used for separating blood components, such that no polysaccharide preparation is present in the collection vessel;
depleting the sample of red blood cells by treating the sample with a hypotonic erythrocyte lysis buffer, for example comprising ammonium chloride, in the absence of any polysaccharide preparation used for separating blood components, to obtain a remaining cell population, such that no polysaccharide preparation is subsequently added to the blood sample to assist with subsequent separation or isolation of blood components, and that the blood sample remains free of any polysaccharide preparation from the time of collection until the remaining cell population is obtained;
expanding the remaining cell population in the sample in an optionally serum-free hematopoietic expansion medium to obtain an expanded cell population that contains 50 % or greater CD71+ cells; and
culturing the expanded cell population in the presence of Oct4, Sox2, and Klf4, and optionally c-MYC, in human embryonic stem cell medium to induce pluripotency.

2. The method of claim 1, wherein the sample has been stored for up to 48 hours prior to use in the method, at a temperature of 3°C to 5°C or on ice.

3. The method of claim 1 or 2, wherein the volume of the blood sample is from 10 µl to 20 µl.

4. The method of any one of claims 1 to 3, wherein the hematopoietic cell is a non-human hematopoietic cell or is a human hematopoietic cell.

5. The method of any one of claims 1 to 4, wherein the expanding occurs for a period of 12 to 16 days.

6. The method of any one of claims 1 to 5, wherein the expanding is performed in a serum-free hematopoietic expansion medium.

7. The method of any one of claims 1 to 6, wherein the expanded cell population, prior to the culturing to induce pluripotency, comprises 20 000 cells or greater.

8. The method of any one of claims 1 to 7, wherein the culturing to induce pluripotency is performed in the presence of feeder cells.

9. The method of any one of claims 1 to 8, wherein the culturing to induce pluripotency comprises including the Oct4, Sox2 and Klf4, and the optional c-MYC, in the human embryonic stem cell medium, or comprises transfecting or transducing the expanded cell population with one or more nucleic acid vectors encoding the Oct4, Sox2 and Klf4, and the optional c-MYC.

10. The method of claim 9, wherein the one or more nucleic acid vectors are viral vectors, episomal vectors, minicircle vectors, or synthesized mRNA.

11. The method of claim 10, wherein the one or more nucleic acid vectors are viral vectors that are adenoviral vectors, baculoviral vectors or Sendai viral vectors.

12. The method of claim 11, wherein the one or more viral vectors are Sendai viral vectors.

13. The method of claim 12, wherein the transducing comprises exposing the expanded cell population to the one or more Sendai viral vectors over a period of 24 hours.

14. The method of any one of claims 1 to 13, further comprising storing the resulting induced pluripotent cells in a cell bank or library.

15. The method of any one of claims 1 to 14, wherein a portion of the blood sample is, prior to the depleting, reserved for characterization, for example DNA sequencing and/or blood serotyping.

## Patentansprüche

1. Verfahren zum Induzieren von Pluripotenz in einer hämatopoetischen Zelle, wobei das Verfahren umfasst:
Bereitstellen einer Blutprobe, wobei das Volumen der Blutprobe von 10 µl bis 250 µl Blut beträgt, gewonnen von einem Subjekt, in Abwesenheit jeglicher Polysaccharidzubereitung, die zum Trennen von Blutkomponenten verwendet wird, so dass keine Polysaccharidzubereitung in dem Sammelgefäß vorliegt;
Abreichern der Probe von roten Blutzellen durch Behandeln der Probe mit einem hypotonen Erythrozyten-Lysepuffer, zum Beispiel umfassend Ammoniumchlorid, in Abwesenheit jeglicher Polysaccharidzubereitung, die zum Trennen von Blutkomponenten verwendet wird, um eine verbleibende Zellpopulation zu erhalten, so dass keine Polysaccharidzubereitung anschließend zu der Blutprobe zugegeben wird, um die anschließende Trennung oder Isolation von Blutkomponenten zu unterstützen, und dass die Blutprobe vom Zeitpunkt des Gewinnens bis zum Erhalten der verbleibenden Zellpopulation frei von jeglicher Polysaccharidzubereitung bleibt;
Expandieren der verbleibenden Zellpopulation in der Probe in einem optional serumfreien hämatopoetischen Expansionsmedium, um eine expandierte Zellpopulation zu erhalten, die 50% oder mehr CD71+-Zellen enthält; und
Kultivieren der expandierten Zellpopulation in Gegenwart von Oct4, Sox2 und Klf4 und optional c-MYC in humanem embryonalem Stammzellmedium, um Pluripotenz zu induzieren.

2. Verfahren gemäß Anspruch 1, wobei die Probe für bis zu 48 Stunden vor Verwendung in dem Verfahren bei einer Temperatur von 3°C bis 5°C oder auf Eis gelagert wurde.

3. Verfahren gemäß Anspruch 1 oder 2, wobei das Volumen der Blutprobe von 10 µl bis 20 µl beträgt.

4. Verfahren gemäß irgendeinem der Ansprüche 1 bis 3, wobei die hämatopoetische Zelle eine nicht-humane hämatopoetische Zelle oder eine humane hämatopoetische Zelle ist.

5. Verfahren gemäß irgendeinem der Ansprüche 1 bis 4, wobei die Expansion für eine Zeitdauer von 12 bis 16 Tagen stattfindet.

6. Verfahren gemäß irgendeinem der Ansprüche 1 bis 5, wobei die Expansion in einem serumfreien hämatopoetischen Expansionsmedium durchgeführt wird.

7. Verfahren gemäß irgendeinem der Ansprüche 1 bis 6, wobei die expandierte Zellpopulation vor dem Kultivieren, um Pluripotenz zu induzieren, 20.000 Zellen oder mehr umfasst.

8. Verfahren gemäß irgendeinem der Ansprüche 1 bis 7, wobei das Kultivieren, um Pluripotenz zu induzieren, in Gegenwart von Feeder-Zellen durchgeführt wird.

9. Verfahren gemäß irgendeinem der Ansprüche 1 bis 8, wobei das Kultivieren, um Pluripotenz zu induzieren, das Einschließen des Oct4, Sox2 und Klf4 und des optionalen c-MYC in dem humanen embryonalen Stammzellmedium umfasst oder Transfizieren oder Transduzieren der expandierten Zellpopulation mit einem oder mehreren Nukleinsäurevektor(en), der/die das Oct4, Sox2 und Klf4 und das optionale c-MYC codiert/codieren, umfasst.

10. Verfahren gemäß Anspruch 9, wobei der eine oder die mehreren Nukleinsäurevektor(en) virale Vektoren, episomale Vektoren, Minicircle-Vektoren oder synthetisierte mRNA ist/sind.

11. Verfahren gemäß Anspruch 10, wobei der eine oder die mehreren Nukleinsäurevektor(en) virale Vektoren sind, die Adenovirusvektoren, Baculovirusvektoren oder Sendai-Virusvektoren sind.

12. Verfahren gemäß Anspruch 11, wobei der eine oder die mehreren viralen Vektoren Sendai-Virusvektoren sind.

13. Verfahren gemäß Anspruch 12, wobei das Transduzieren das Aussetzen der expandierten Zellpopulation an den einen oder die mehreren Sendai-Virusvektoren über einen Zeitraum von 24 Stunden umfasst.

14. Verfahren gemäß irgendeinem der Ansprüche 1 bis 13, welches weiterhin das Lagern der resultierenden induzierten pluripotenten Zellen in einer Zellbank oder Bibliothek umfasst.

15. Verfahren gemäß irgendeinem der Ansprüche 1 bis 14, wobei ein Teil der Blutprobe vor der Abreicherung zur Charakterisierung, zum Beispiel DNA-Sequenzierung und/oder Blut-Serotypisierung, reserviert wird.

## Revendications

1. Procédé pour induire une pluripotence dans une cellule hématopoïétique, le procédé comprenant :
la fourniture d'un échantillon sanguin, dans lequel le volume de l'échantillon sanguin est de 10 µl à 250 µl de sang prélevé sur un sujet en l'absence de toute préparation polysaccharidique utilisée pour séparer les composants sanguins, de sorte qu'aucune préparation polysaccharidique n'est présente dans le récipient de prélèvement ;
la déplétion de l'échantillon de globules rouges en traitant l'échantillon avec un tampon hypotonique de lyse des érythrocytes, par exemple comprenant du chlorure d'ammonium, en l'absence de toute préparation polysaccharidique utilisée pour séparer les composants sanguins, afin d'obtenir une population de cellules résiduelles, de sorte qu'aucune préparation polysaccharidique n'est par la suite ajoutée à l'échantillon sanguin pour faciliter la séparation ou l'isolement ultérieur(e) des composants sanguins et de sorte que l'échantillon sanguin reste exempt de toute préparation polysaccharidique du moment du prélèvement jusqu'à l'obtention de la population de cellules résiduelles ;
l'expansion de la population de cellules résiduelles dans l'échantillon dans un milieu d'expansion hématopoïétique facultativement sans sérum afin d'obtenir une population de cellules expansées contenant 50 % ou plus de cellules CD71+ ; et
la culture de la population de cellules expansées en présence d'Oct4, de Sox2 et de Klf4, et facultativement de c-MYC, dans du milieu de cellules souches d'embryon humain, afin d'induire une pluripotence.

2. Procédé selon la revendication 1, dans lequel l'échantillon est conservé jusqu'à 48 heures à une température de 3 °C à 5 °C ou sur de la glace avant d'être utilisé dans le procédé.

3. Procédé selon la revendication 1 ou 2, dans lequel le volume de l'échantillon sanguin est de 10 µl à 20 µl.

4. Procédé selon l'une quelconque des revendications 1 à 3, dans lequel la cellule hématopoïétique est une cellule hématopoïétique non humaine ou une cellule hématopoïétique humaine.

5. Procédé selon l'une quelconque des revendications 1 à 4, dans lequel l'expansion se déroule sur une période de 12 à 16 jours.

6. Procédé selon l'une quelconque des revendications 1 à 5, dans lequel l'expansion est réalisée dans un milieu d'expansion hématopoïétique sans sérum.

7. Procédé selon l'une quelconque des revendications 1 à 6, dans lequel la population de cellules expansées comprend 20 000 cellules ou plus avant la culture pour induire une pluripotence.

8. Procédé selon l'une quelconque des revendications 1 à 7, dans lequel la culture pour induire une pluripotence est réalisée en présence de cellules nourricières.

9. Procédé selon l'une quelconque des revendications 1 à 8, dans lequel la culture pour induire une pluripotence comprend l'inclusion d'Oct4, de Sox2 et de Klf4, et de c-MYC facultatif, dans le milieu de cellules souches d'embryon humain, ou comprend la transfection ou la transduction de la population de cellules expansées avec un ou plusieurs vecteurs d'acides nucléiques codant pour Oct4, Sox2 et Klf4, et c-MYC facultatif.

10. Procédé selon la revendication 9, dans lequel le ou les vecteurs d'acides nucléiques sont des vecteurs viraux, des vecteurs épisomiques, des vecteurs minicercles ou l'ARNm synthétisé.

11. Procédé selon la revendication 10, dans lequel le ou les vecteurs d'acides nucléiques sont des vecteurs viraux qui sont les vecteurs adénoviraux, les vecteurs baculoviraux ou les vecteurs du virus Sendai.

12. Procédé selon la revendication 11, dans lequel le ou les vecteurs viraux sont les vecteurs du virus Sendai.

13. Procédé selon la revendication 12, dans lequel la transduction comprend l'exposition de la population de cellules expansées à un ou plusieurs vecteurs du virus Sendai sur une période de 24 heures.

14. Procédé selon l'une quelconque des revendications 1 à 13, comprenant en outre la conservation des cellules pluripotentes induites obtenues dans une banque ou une bibliothèque de cellules.

15. Procédé selon l'une quelconque des revendications 1 à 14, dans lequel une partie de l'échantillon sanguin est, avant déplétion, réservé à la caractérisation, par exemple au séquençage de l'ADN et/ou au sérotypage sanguin.
